# EUROPEAN PATENT APPLICATION

(11) **EP 4 650 360 A2**
(43) Date of publication of application: **19.11.2025**
(21) Application number: 25205862.3
(22) Date of filing: 17.08.2018
(51) Int. Cl.: C07K 1/28

(54) **IMAGE CAPILLARY ISOELECTRIC FOCUSING TO ANALYZE PROTEIN VARIANTS IN A SAMPLE MATRIX**

(30) Priority: 18.08.2017 US 201762547602 P
(62) Divisional of application: 18845630.5
(71) Applicant: Regeneron Pharmaceuticals, Inc., Tarrytown, NY 10591 (US)
(72) Inventor: RAMBHADRAN, Anu, Tarrytown, NY 10591 (US); CUI, Peng, Tarrytown, NY 10591 (US); RYAN, Clare, Tarrytown, NY 10591 (US); BIGWARFE, Paul, Tarrytown, NY 10591 (US); LASTRO, Michele, Tarrytown, NY 10591 (US); MA, Junyu, Tarrytown, NY 10591 (US); LU, Kun, Tarrytown, NY 10591 (US)
(74) Representative: Carpmaels & Ransford LLP

(57) **Abstract**

Embodiments of the present disclosure are directed to methods, systems, devices and kits corresponding to a method for analyzing charge variants of a protein such as vascular endothelial growth factor VEGF-Trap.

## Description

### CROSS-REFERENCE TO RELATED APPLICATIONS

This application claim priority to U.S. Provisional Application Serial No. 62/547,602 filed on August 18, 2017, which is hereby incorporated by reference in its entirety.

### SEQUENCE LISTING

The instant application contains a Sequence Listing which has been submitted in ASCII format via EFS-Web and is hereby incorporated by reference in its entirety. Said ASCII copy, created on August 15, 2018 is named REGE-005_001 WO_ST25.txt and is 4,214 bytes in size.

### FIELD OF THE DISCLOSURE

The field of the present disclosure is directed to methods and systems for analyzing charge variants of proteins such as VEGF Trap in a sample matrix.

### BACKGROUND

The analysis of charge variants is often desirable for various proteins used as biopharmaceuticals because such changes can affect drug activity, stability, and in some cases, patient safety. Conventional methods employed in the industry for identifying and characterizing charge variants include ion-exchange chromatography, isoelectric focusing gel electrophoresis, and capillary isoelectric focusing. Image capillary isoelectric focusing has been found to be useful due to its high resolution, reduced sample volume, and fast run times. Accordingly, methods and systems using image capillary isoelectric focusing to determine charge variants for proteins such as VEGF Trap would be beneficial.

### SUMMARY OF THE INVENTION

Described herein are methods and systems for charge variant analysis of various proteins. For example, the methods and systems can be used to analyze charge variants of VEGF Trap. Instead of reporting charge variant distribution by grouping bands 3-9 in an isoelectric focusing gel, which is the currently approved method for analyzing VEGF Trap, the methods and systems described here generally use image capillary isoelectric focusing to report charge heterogeneity in terms of percentages of charge variant isoforms, and groups them into three different regions of the electropherogram. This reporting approach may be more sensitive to changes that occur in the isoforms of VEGF Trap samples.

As noted above, embodiments of the present disclosure are directed to methods, systems and devices for determining charge variants of a protein, and in particular, an image capillary isoelectric focusing (iCIEF) assay to assess charge variance of proteins such as vascular endothelial growth factor (VEGF) blocker, hereinafter referred to as "VEGF-Trap." iCIEF is an alternative for the currently approved Isoelectric Focusing (IEF) method for VEGF-Trap charge variant analysis.

Embodiments of iCIEF correspond to techniques which separate protein charge variants based upon their isoelectric point (pI). For example, in some embodiments, a protein sample is loaded onto a separation capillary comprising a mixture of carrier ampholyte (e.g., Pharmalyte^{™}), methylcellulose, and a stabilizing additive (i.e. urea). A voltage is applied for a predetermined period of time resulting in the carrier ampholyte forming a pH gradient within the capillary. In some embodiments, the voltage is applied for a second, longer period of time corresponding to a "focusing" time. This results in the protein charge variants migrating within the capillary until reaching a point where the overall charge of the variants is neutral (i.e., their pI).

In such embodiments, the capillary tube (which is coated with fluorocarbon (FC)) is coupled to a digital (e.g., CCD) camera which enables direct detection and quantitation of the protein charge variants. Specifically, after the focusing time, the CCD camera is configured to image the capillary tube (preferably in real time) to detect the protein within the capillary. Detection, in some embodiments, occurs at a wavelength of approximately 280 nm. Parameters in this technique include:
brand and concentration of ampholyte,
type and concentration of additives used, and
focusing time and sample concentration.

Aggregation and precipitation of the protein within the capillary is detrimental to the reproducibility of the electropherogram. To this end, additives, such as urea, may be used to help stabilize and solubilize the protein as it is focused.

Accordingly, in some embodiments, a method for analyzing charge variants of vascular endothelial growth factor VEGF-Trap is provided and includes loading a protein sample onto a separation capillary having a mixture of at least a carrier ampholyte, methylcellulose, and a stabilizing additive, applying a first voltage for a first predetermined period of time such that the carrier ampholyte forms a pH gradient within the capillary, applying a second voltage for a second predetermined period of time to focus the migration of charge variants of the protein to their respective pI, and detecting and quantifying charge variants of the protein.

In such embodiments, detecting and quantifying charge variants comprises measuring the absorbance for a plurality of charge variant isoforms, segregating the plurality of charge variant isoforms into isolated regions comprising at least a first acid/acidic region (R1), a second neutral region (R2), and a third base/basic region (R3), and determining a percentage of charge variant isoforms falling within in each of regions R1, R2 and R3.

For such embodiments, image analysis for detecting and quantification can be according to conventional methods and systems (e.g., image analysis software.

In the embodiments summarized above, one and/or another of the following additional features/functionality may be included (resulting yet in further inventive embodiments), however, it should be pointed out that any one or more of these features may be different and yet be within the scope of the present invention - the list provided below is but one embodiment:
detecting and quantifying of charge variants are performed;
detection of charge variants occurs at a wavelength of approximately 280 nm;
the concentration of protein loaded onto the capillary tube is approximately 2.0 mg/ml;
the stabilizing additive comprises urea;
the amount of urea in the mixture comprises approximately 2M;
the mixture includes approximately 0.35% methylcellulose;
the first voltage comprises 1500 V;
the first predetermined time is approximately 1 minute;
the second voltage comprises 3000 V;
the second predetermined time is approximately 7 minute;
the concentration of protein loaded onto the capillary tube is between approximately 1.0 and 8.0 mg/ml;
the amount of urea in the mixture is greater than 0M and less than approximately 8M;
the mixture includes between approximately 0.01% and approximately 0.35% methylcellulose, or any intervening range;
the first voltage is between approximately 1V and about 3000 V, or any intervening range;
the first predetermined time is between approximately 1 second and approximately 5 minutes, or any intervening range;
the second voltage is between about 1V and approximately 3000 V, or any intervening range; and/or
the second predetermined time is between approximately 1 minute and approximately 14 minutes, or any intervening range.

In some embodiments, an iCIEF capillary tube configured for use in a charge variant analysis of VEGF-Trap is provided and includes a capillary tube configured to receive a protein, and configured with a mixture of carrier ampholyte, methylcellulose, and a stabilizing additive. The capillary tube may also include a fluorocarbon coating.

In some embodiments, an iCIEF kit configured for use in a charge variant analysis of VEGF-Trap is provided and includes one or more capillary tubes configured to receive a protein, and configured with a mixture of carrier ampholyte, methylcellulose, and a stabilizing additive, wherein the capillary tube includes a fluorocarbon coating.

### BRIEF DESCRIPTION OF THE DRAWINGS

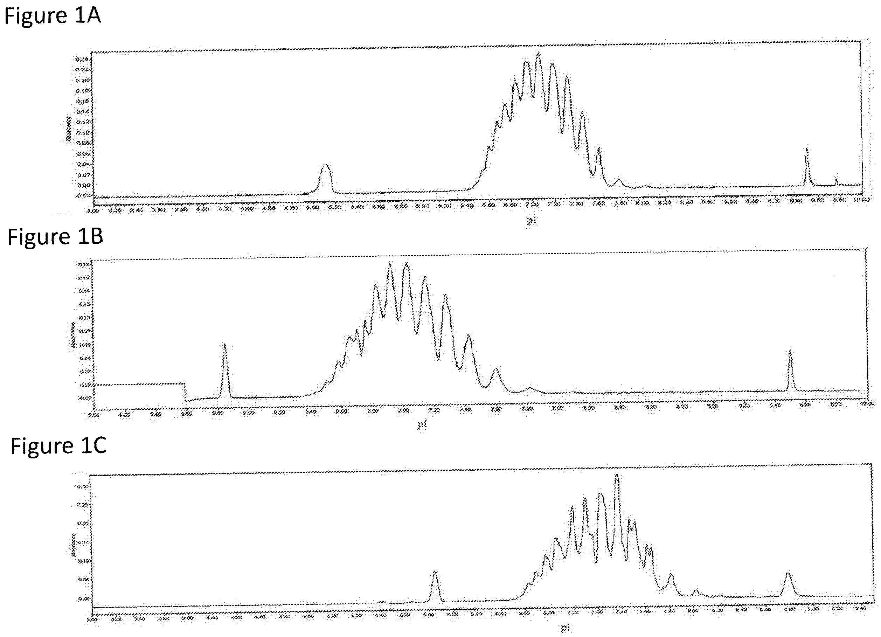

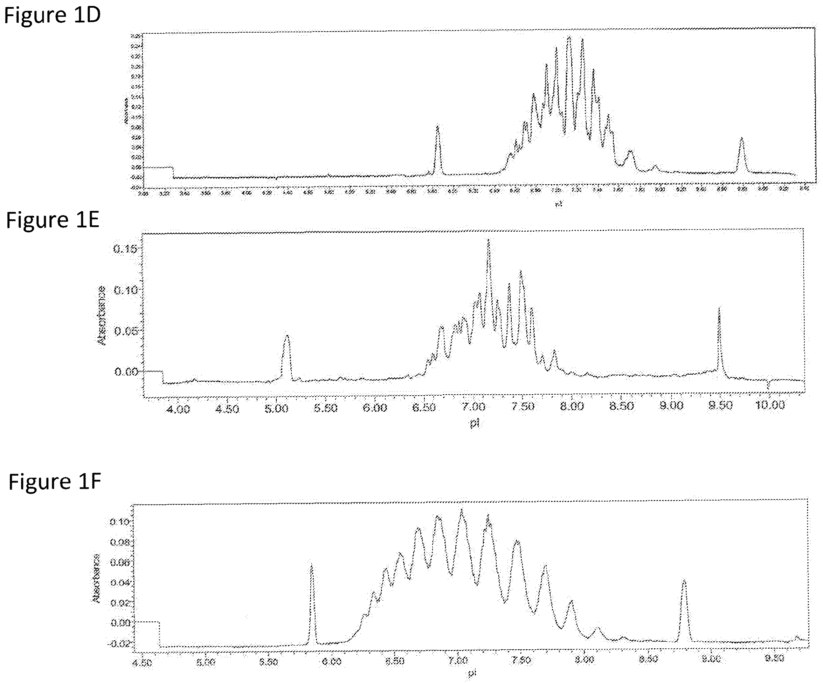

Figures 1A-1F depict electropherograms of VEGF Trap using different ampholytes. In Figure 1A, the ampholyte is Pharmalyte^{™} having a pI ranging from 3-10; in Figure 1B, the ampholyte is a combination of Pharmalyte^{™} with pI ranging from 5-8 and Pharmalyte^{™} having a pI ranging from 8-10.5; in Figure 1C, the ampholyte is Servalyt^{™} having a pI ranging from 2-9; in Figure 1D, the ampholyte is Servalyt^{™} having a pI ranging from 4-9; in Figure 1E, the ampholyte is Biolyte^{™} having a pI ranging from 3-10; and Figure 1F, the ampholyte is a combination of Pharmalyte^{™} having a pI ranging from 3-10 and Pharmalyte^{™} having a pI ranging from 6.7 to 7.
Figure 2A-2E compare the electropherograms of VEGF Trap at varying urea concentrations.
Figure 3 compares the electropherogram of VEGF Trap obtained using isoelectric focusing and image capillary isoelectric focusing methods.
Figures 4A-4G illustrate VEGF Trap OFFGEL fraction analysis (fractions #5-#10) using isoelectric focusing and image capillary isoelectric focusing methods.
Figures 5A-5F illustrate electropherograms of VEGF Trap RS spiked with VEGF Trap OFFGEL fractions (fractions #5-#10) analyzed in Figs. 4A-4G.
Figure 6 compares the electropherogram of a blank and VEGF Trap spiked with an independent marker.
Figure 7A shows the image capillary isoelectric focusing electropherogram of a VEGF Trap RS sample with Regions 1, 2, and 3 assigned.
Figure 7B illustrates the difference in electropherogram reporting between the isoelectric focusing method and image capillary isoelectric focusing method.
Figure 8 provides data obtained using image capillary isoelectric focusing relating to VEGF Trap stability.
Figure 9 provides stability data obtained using image capillary isoelectric focusing performed on forcibly degraded VEGF Trap samples.
Figures 10A-10C show the statistical analysis of image capillary isoelectric focusing data provided in Figure 9.
Figure 11 provides data relating to linearity of the image capillary isoelectric focusing method.
Figure 12 compares image capillary isoelectric electropherograms for three samples of ampholyte.
Figures 13A-13D show the statistical analysis of image capillary isoelectric focusing data obtained from historical VEGF Trap samples.

### DETAILED DESCRIPTION OF THE INVENTION

Described herein are methods and systems for charge variant analysis of various proteins such as VEGF Trap. VEGF Trap is a fusion protein comprising the sequence shown in Table 1 Instead of reporting charge variant distribution by grouping bands 3-9 in an isoelectric focusing gel, which is the currently approved method for analyzing VEGF Trap, the methods and systems described here generally use image capillary isoelectric focusing to report charge heterogeneity in terms of percentages of charge variant isoforms, and groups them into three different regions of the electropherogram. This reporting approach may be more sensitive to changes that occur in the isoforms of VEGF Trap samples, as previously mentioned.

**Table 1 - VEGF Trap sequence**

| **Protein** | **Sequence** | **SEQ ID NO** |
|---|---|---|
| VEGF Trap | SDTGRPFVEMYSEIPEIIHMTEGRELVIPCRVTSPNITVTLK KFPLDTLIPDGKRIIWDSRKGFIISNATYKEIGLLTCEATVN GHLYKTNYLTHRQTNTIIDVVLSPSHGIELSVGEKLVLNC TARTELNVGIDFNWEYPSSKHQHKKLVNRDLKTQSGSEM KKFLSTLTIDGVTRSDQGLYTCAASSGLMTKKNSTFVRV HEKDKTHTCPPCPAPELLGGPSVFLFPPKPKDTLMISRTPE VTCVVVDVSHEDPEVKFNWYVDGVEVHNAKTKPREEQ YNSTYRVVSVLTVLHQDWLNGKEYKCKVSNKALPAPIE KTISKAKGQPREPQVYTLPPSRDELTKNQVSLTCLVKGFY PSDIAVEWESNGQPENNYKTTPPVLDSDGSFFLYSKLTVD KSRWQQGNVFSCSVMHEALHNHYTQKSLSLSPG | 1 |

The following acronyms are used throughout the present disclosure:
iCIEF - Imaging Capillary Isoelectric Focusing
IEF - Isoelectric Focusing
pI - Isoelectric point
RS - Reference Standard
DS - Drug substance
DSI - Drug Substance Intermediate
FDS - Formulated Drug Substance.

The methods for analyzing charge variants of VEGF Trap generally include loading a protein sample onto a separation capillary comprising a mixture of at least a carrier ampholyte, methylcellulose, and a stabilizing additive, applying a first voltage for a first predetermined period of time such that the carrier ampholyte forms a pH gradient within the capillary, applying a second voltage for a second predetermined period of time to focus the migration of charge variants of the protein within the capillary such that the overall charge of the variants is neutral, and detecting and quantifying charge variants of the protein.

The separation capillary may be loaded with VEGF Trap at a concentration ranging from about 0.5 mg/mL to about 2 mg/mL. For example, the separation capillary may be loaded with VEGF Trap at a concentration of about 0.5 mg/mL, about 1.0 mg/mL, about 1.5 mg/mL, or about 2 mg/mL. In some embodiments, the separation capillary is loaded with VEGF Trap at a concentration of about 1.0 mg/mL.

The amount of methylcellulose in the mixture may range from about 0.01% to about 0.35%. For example, the amount of methylcellulose in the mixture may be about 0.01%, about 0.05%, about 0.10%, about 0.15%, about 0.20%, about 0.25%, about 0.30%, or about 0.35%. In some embodiments, the amount of methylcellulose in the mixture is about 0.35%.

With respect to the first voltage, it may range from approximately 1 V to approximately 3000 V. For example, the first voltage may be about 1 V, about 100 V, about 500 V, about 1000 V, about 1500 V, about 2000 V, about 2500 V, or about 3000 V. In some embodiments, the first voltage is about 1500 V.

The second voltage may also range from approximately 1 V to about 3000 V. For example, the second voltage may be about 1 V, about 100 V, about 500 V, about 1000 V, about 1500 V, about 2000 V, about 2500 V, or about 3000 V. In some embodiments, the second voltage is about 3000 V.

The first predetermined time may range from about 1 second to about 5 minutes. For example, the first predetermined time may be about 1 second, about 10 seconds, about 20 seconds, about 30 seconds, about 40 seconds, about 50 seconds, about 1 minute (60 seconds), about 1.5 minutes (90 seconds), about 2 minutes (120 seconds), about 2.5 minutes (150 seconds), about 3 minutes (180 seconds), about 3.5 minutes (210 seconds), about 4 minutes (240 seconds), about 4.5 minutes (270 seconds), or about 5 minutes (300 seconds). In some embodiments, the first predetermined time is about 1 minute (60 seconds).

The second predetermined time may range from about 1 minute to about 14 minutes. For example, the second predetermined time may be about 1 minute, about 2 minutes, about 3 minutes, about 4 minutes, about 5 minutes, about 6 minutes, about 7 minutes, about 8 minutes, about 9 minutes, about 10 minutes, about 11 minutes, about 12 minutes, about 13 minutes, or about 14 minutes. In some embodiments, the second predetermined time is about 7 minutes.

Any suitable additive may be employed in the mixture. In some embodiments, it may be beneficial to use urea as the additive. For example, 2M urea may be beneficial to include in the mixture. Various reagents (ampholytes) may also be included in the mixture, as further detailed below. In one embodiment, VEGF Trap is loaded into a capillary at a concentration of 1.0 mg/mL, and analyzed using an image capillary isoelectric focusing method that employs a mixture of 0.35% methylcellulose, 2M urea, and 3% ampholyte having a pI of 3-10.

***Reagents and Equipment.*** Table 2 below lists reagents (ampholytes) and equipment used according to some embodiments of the present disclosure. Examples performed utilize an iCE3 (ProteinSimple^{®}) charge variant analyzer. Unless otherwise indicated, VEGF-Trap Reference Standard (RSVITV-5), was used as a test article during method development and characterization.

**Table 2 - Example Reagents and other components used**

| **Sample Reagent** |
|---|
| Pharmalyte^{™} 3-10 |
| Servalyt^{™} 4-9 |
| Servalyt^{™} 2-9 |
| Pharmalyte^{™} 5-8 |
| Pharmalyte^{™} 8-10.5 |
| Pharmalyte^{™} 6.7-7.7 |
| Biolyte^{™} 3-10, 40% |
| Urea |
| Methylcellulose |
| pI marker (5.12, 7.05, 7.65) |
| ProteinSimple^{®} iCE3 |

### Example for at least some of the embodiments:

Ampholyte screening was initially performed based upon a pI range and source of ampholytes. Four ampholytes, each covering a unique pI range, were procured from three different sources. The ampholytes were analyzed using the following starting:
2.0 mg/mL protein concentration,
2 M urea,
0.35% methylcellulose,
1 minute of pre-focusing at 1500 V, and
7 minutes of focusing at 3000 V.

Figures 1A-F illustrate the electropherogram obtained using six ranges of ampholytes with the iCE3 charge analyzer. The following ampholyte ranges were used:

| | |
|---|---|
| Figure A - 3-10 Pharmalyte | Figure B - 5-8/8-10.5 combo Pharmalyte |
| Figure C - 2-9 Servalyt | Figure D - 4-9 Servalyt |
| Figure E - Bio-Lyte 3-10, 40% | Figure F - 3-Blend of 3-10 and 6.7-7.7 Pharmalyte |

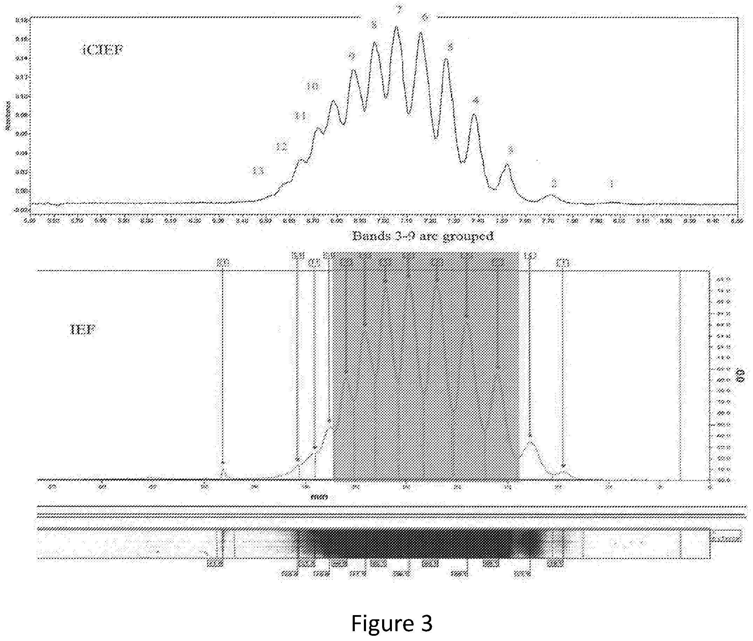

**Protein Selected.** Ampholytes ranging from pI 3-10 were chosen as the overall profile of the iCIEF electropherogram since they most closely resembled the electropherogram from the currently approved charge variant analysis procedure for VEGF-Trap (see, e.g., IEF image shown in Figure 3).

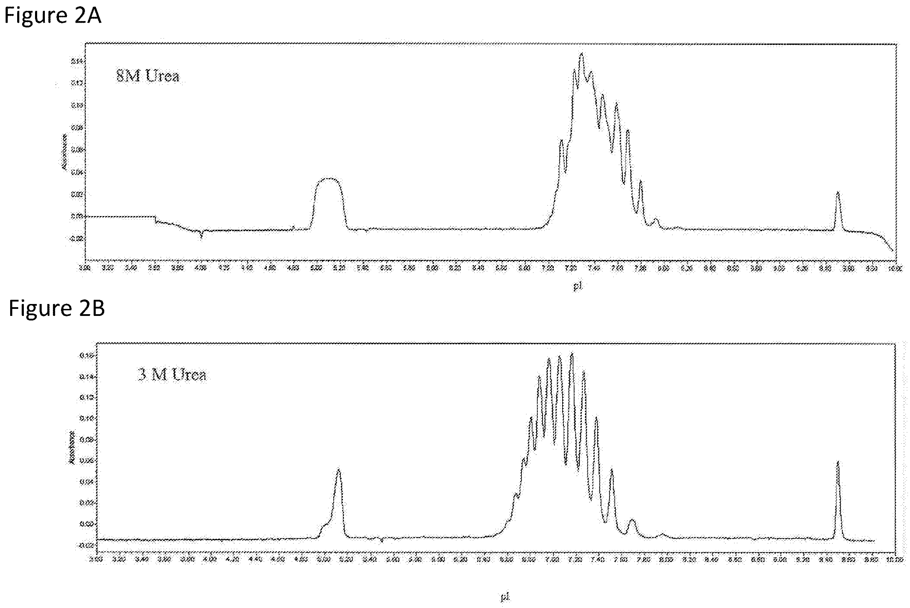

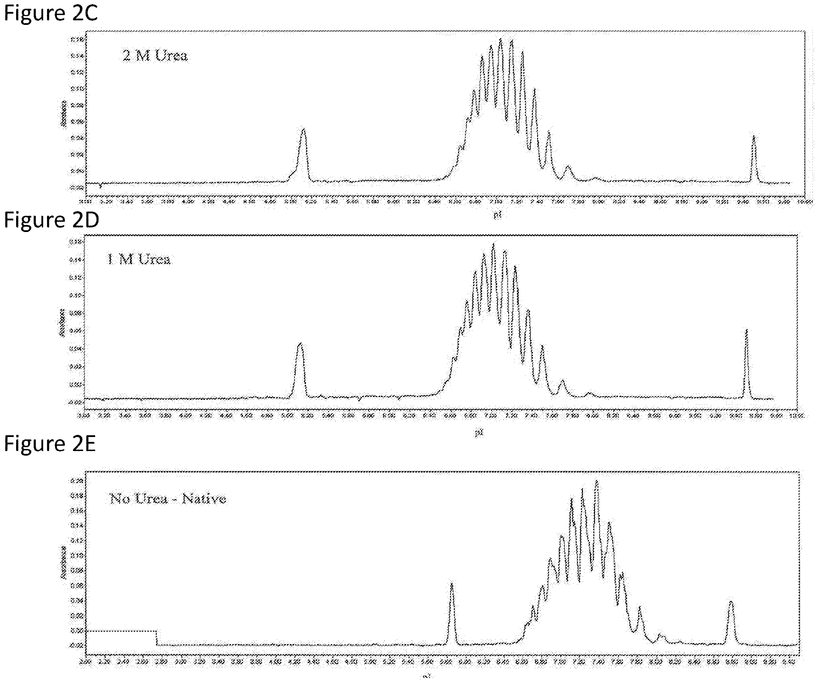

**Urea optimization.** Method optimization, according to some embodiments, also included varying urea concentration (from absence of urea up to 8M). Figures 2A-2E illustrate the effect of such varying urea concentration on the VEGF-Trap RSVITV-5 sample electropherogram. While reduction in urea concentration typically improves resolution, it was found that increased urea (8M) lead to a decrease in resolution. However, VEGF-Trap resolved under native conditions (no urea) had similar issues of decreased resolution and lacked reproducibility. Accordingly, the overall peak pattern and resolution was comparable to each other for VEGF-Trap when separated using 1-3 M urea concentration.

Further experiments were conducted using 2 M urea to optimize the ampholyte 3-10 concentration and protein concentration. Figure 3 illustrates the electropherograms obtained using 2 M Urea, 0.35% Methyl Cellulose and 3% 3-10 ampholyte at 1.0 mg/mL protein concentration. The iCIEF electropherogram was compared to another electropherogram and a tentative peak assignment was made (Figure 3).

**Method Characterization.** The overall charge profile and the pattern of peaks obtained using the iCIEF, assay method was comparable to the IEF band profile (as shown in Figures 2A-2E). However, to further understand and bridge the banding pattern of the IEF assay method to the peak pattern obtained using iCIEF analysis, OFFGEL fractionation of VEGF-Trap sample was undertaken. The individual charge variant fractions obtained from the OFFGEL electrophoresis were analyzed using IEF and iCIEF assay methods. An Agilent 3100 OFFGEL Fractionator was used to fractionate VEGF-Trap Reference Standard according to its isoelectric points, and the separated isoforms recovered as liquid fractions were analyzed using the two methods, IEF and iCIEF. VEGF-Trap RS was fractionated using Immobilized pH Gradient strips (IPG strips) with a pH 6-9 that covers the pI range of charge variants observed for VEGF-Trap. A detailed set up of the experiment and separation is as follows:
1) A stock solution was prepared by combining 2.3 mL 50% glycerol, 230.4 µl of IPG buffer (pH 6-11), and 16.64 ml water to produce a total volume of 19.2 ml.
2) VEGF Trap solution was prepared by adding 73 µg VEGF (4.2 mg) to 12 mL stock solution and 3 mL water.
3) IPG strip rehydration solution was prepared in excess by combining 1.15 mL of water with 4.6 mL of stock solution.
4) IPG gel strips, pH range of 6-9 (24 cm), were arranged in every other lane of the two instrument trays, and 24 well frames were snapped in place over them. The standard OFFGEL kit protocol (*see* OFFGEL user manual: Agilent 3100 OFF GEL Fractionator Kit quick Start Guide, 5th Edition Sep 2010) was used for strip rehydration, antibody loading, and loading of the trays onto the instrument.
5) A platform temperature of 20°C was used. The standard instrument protein focusing method for a 24 well setup was run using a constant current of 50 µA with a max voltage setting of 8000 V and a max power setting of 200 mW.
6) After 34 h of fractionation, the run was stopped and like well numbers from each lane for wells 3 to 12 were pooled, then exchanged into water, and concentrated approximately 5-fold prior to analysis.
7) Antibody quantities in each fraction were determined by measuring the absorbance at 280 nm with extinction coefficient of 1.15 on a Nanodrop. One instrument to determine the concentration of the fraction and then multiplying the volume of the fraction by the concentration.

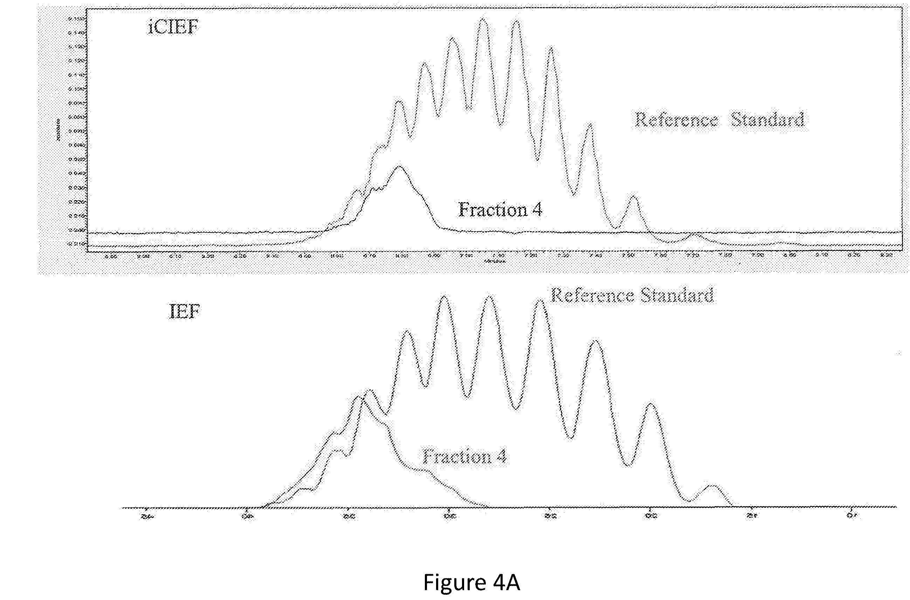

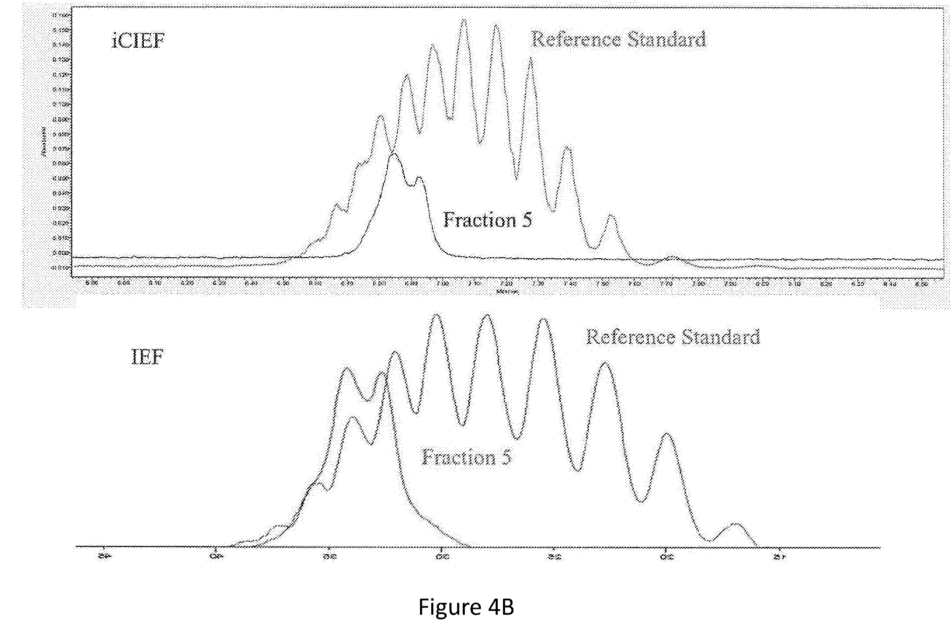

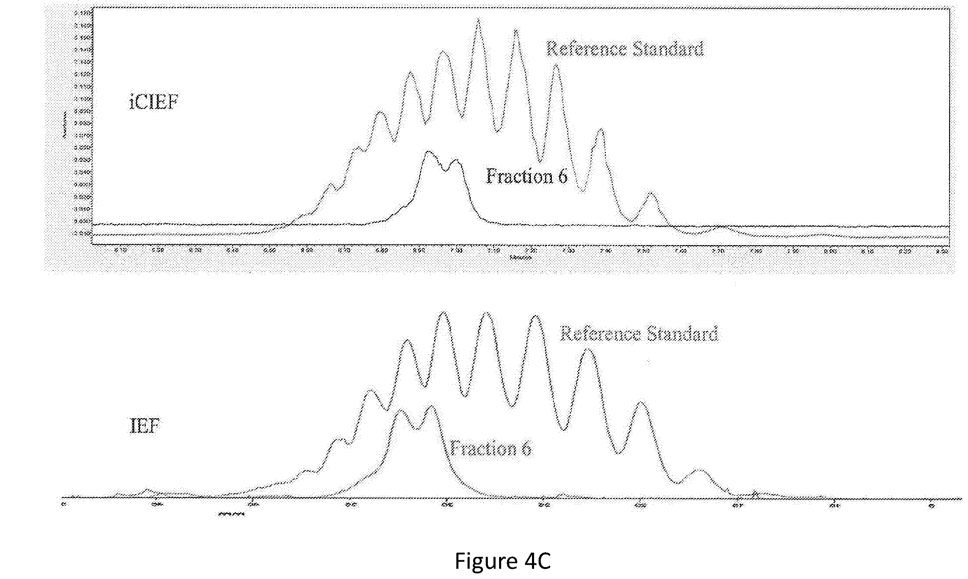

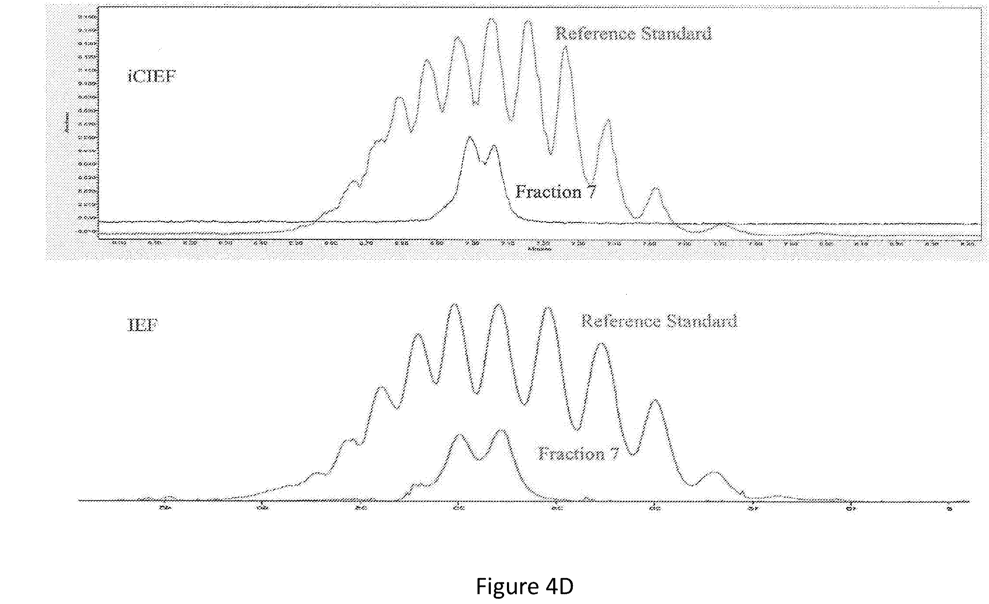

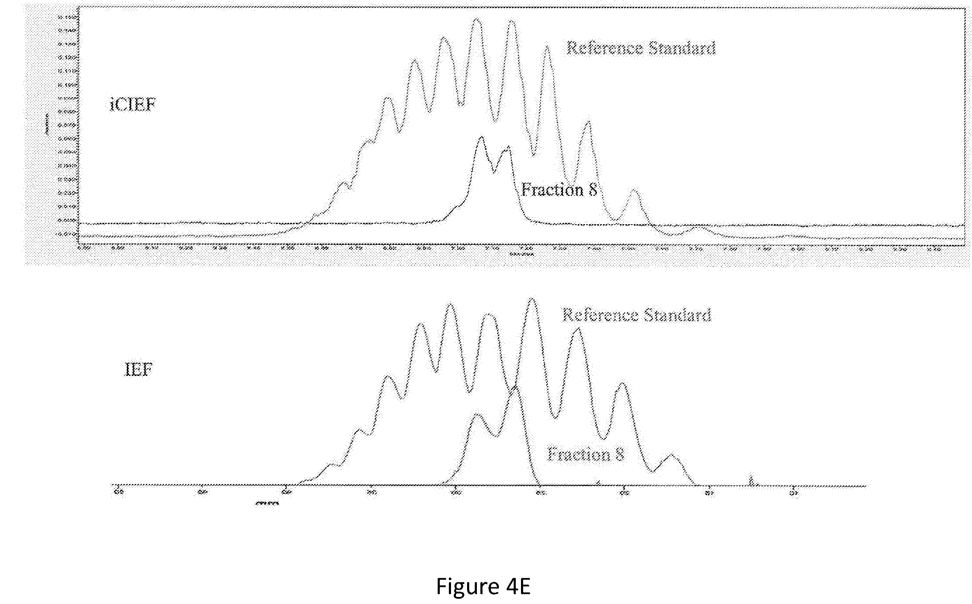

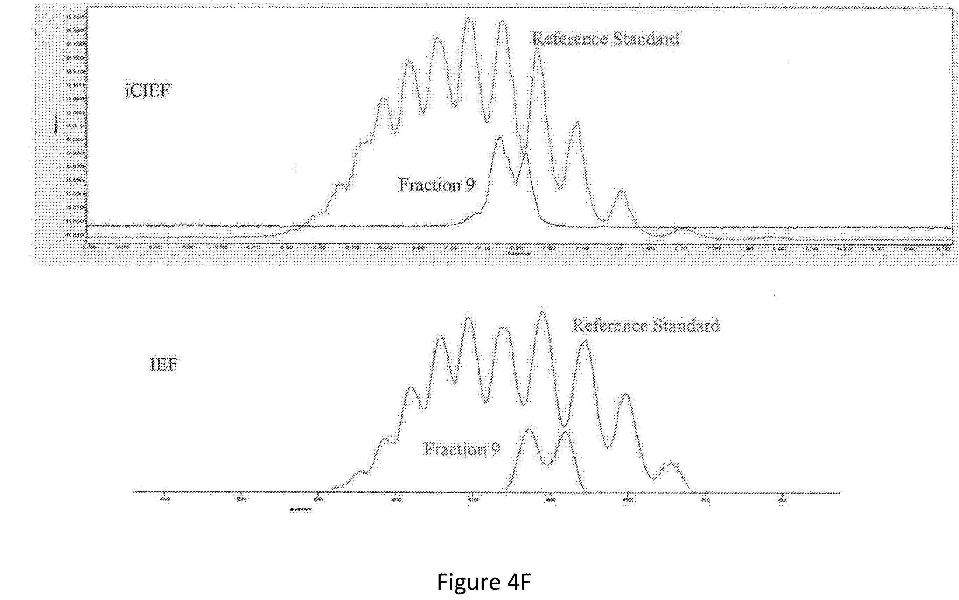

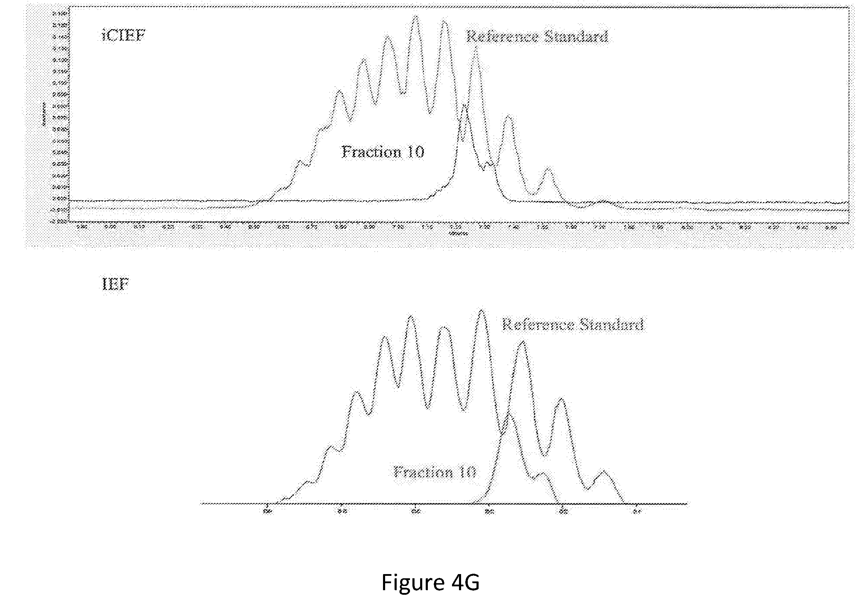

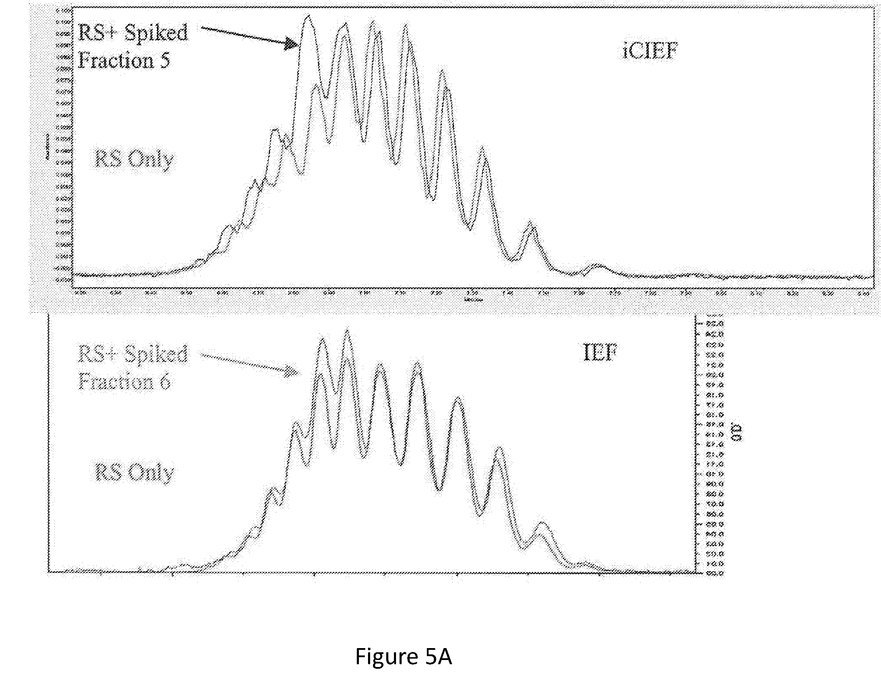

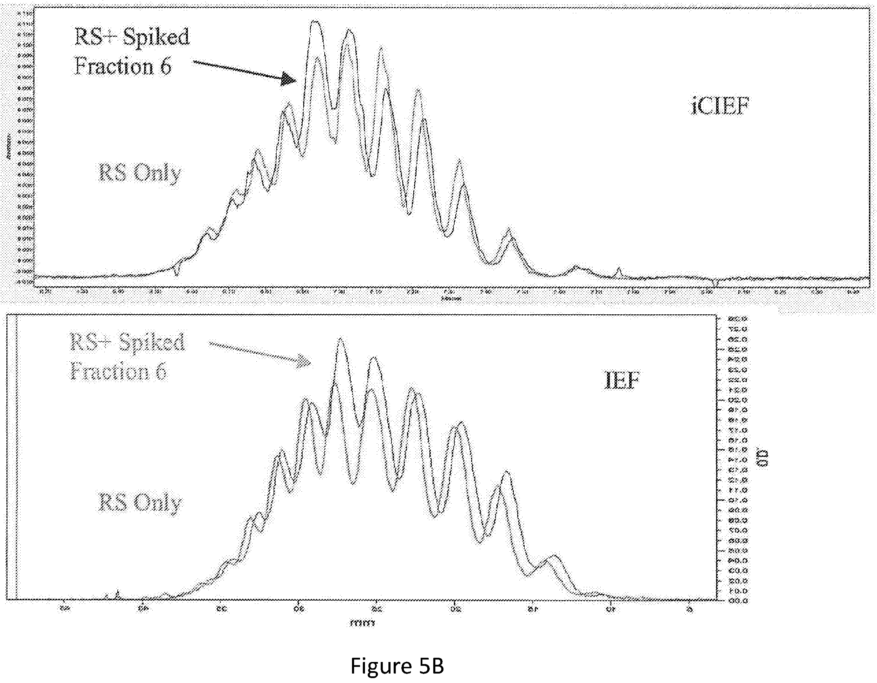

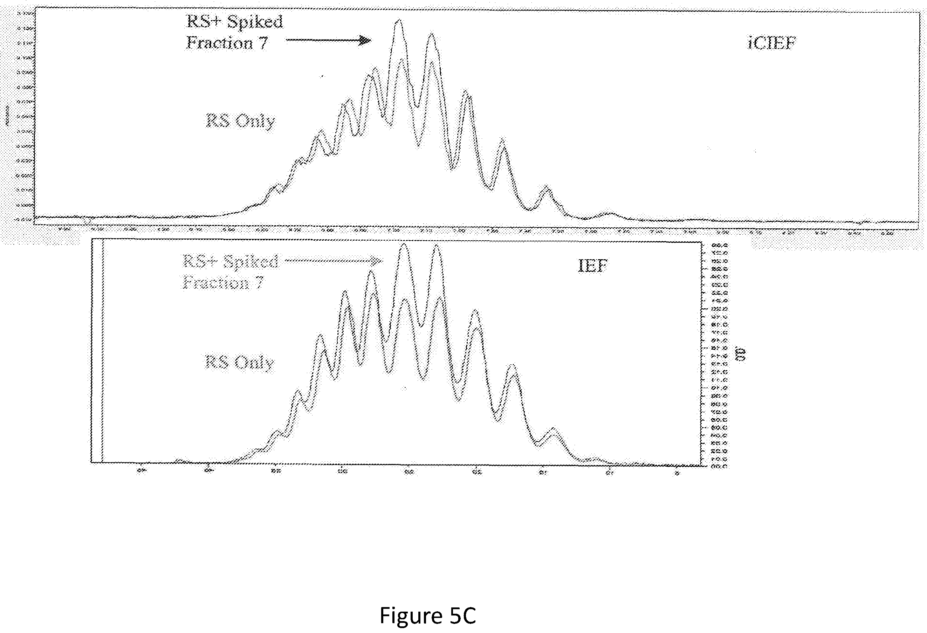

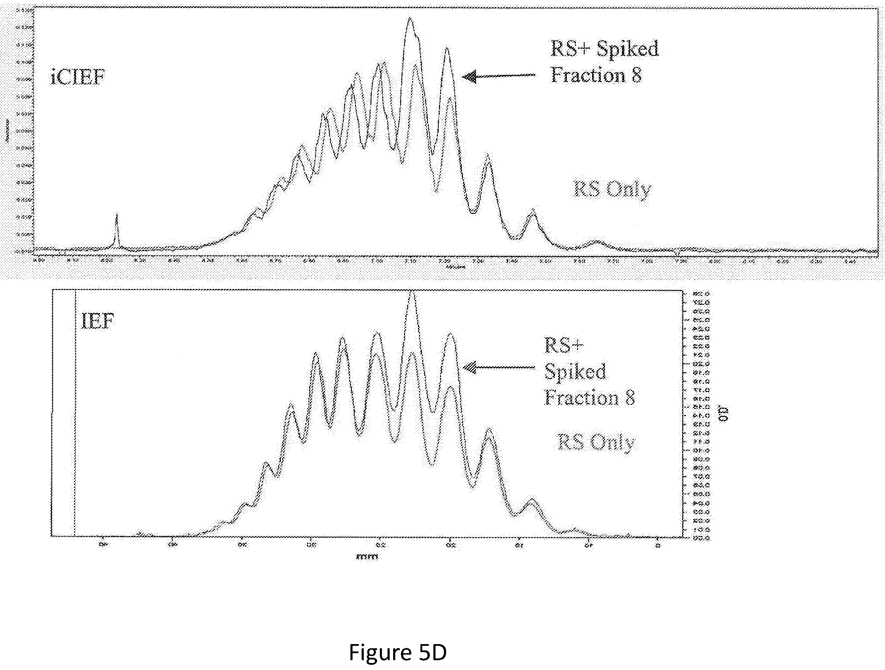

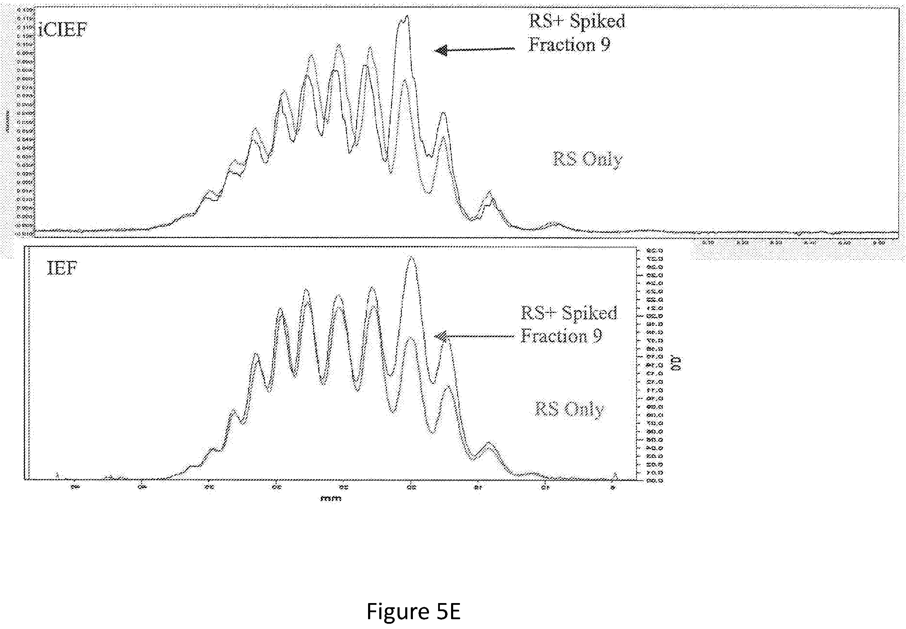

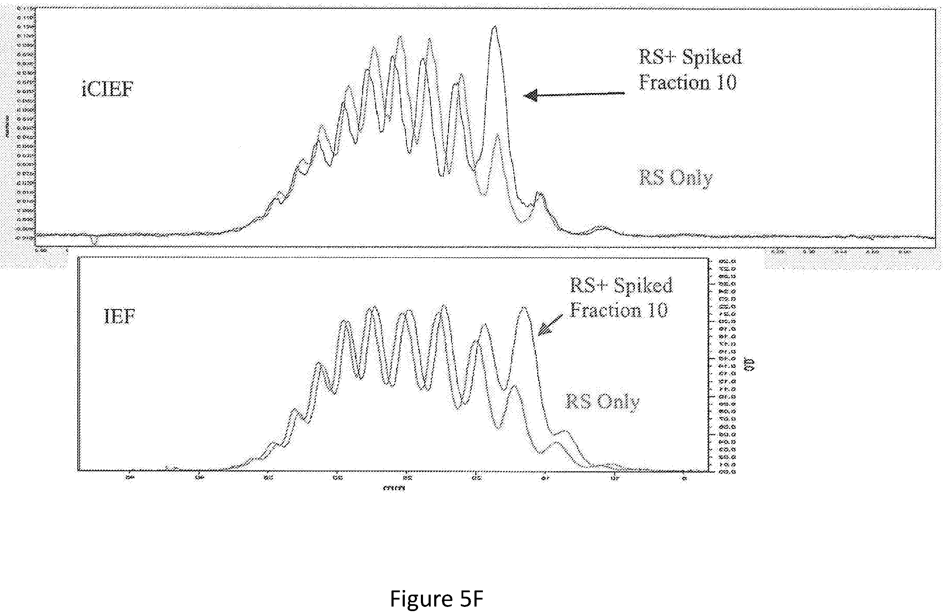

Briefly, 4.2 mg of VEGF-Trap RS was fractionated using 12 IPG strips for 32 hours; the individual fractions from each strip corresponding to the same pI range were pooled and quantified after dialysis. From the fractionation, a total of seven fractions (Fractions 4-10) which had sufficient recovery were analyzed using IEF and iCIEF. The fractions were analyzed two ways: Individual analysis of OFFGEL fractions (4-10) using IEF and iCIEF assay methods (see Figure 4) and spiking of the OFFGEL fractions (5-10) into the VEGF-Trap RS followed by analysis of the spiked samples using IEF and iCIEF (Figure 5). Table 3 lists the fractions and the corresponding amount recovered from the OFFGEL Fractionation study.

**Table 3**

| OFFGEL fraction No. | Protein Cone. (mg/mL) | Volume Recovered (µL) |
|---|---|---|
| 3* | 1.229 | 15 |
| 4 | 1.628 | 98 |
| 5 | 1.884 | 104 |
| 6 | 2.909 | 113 |
| 7 | 3.498 | 124 |
| 8 | 3.56 | 124 |
| 9 | 3.326 | 124 |
| 10 | 2.098 | 110 |
| 11* | 1.235 | 94 |
| 12* | 2.574 | 15 |

In addition to analyzing the OFFGEL fractions independently using IEF and iCIEF, fractions (5-10) which yielded higher recovery were spiked at a ratio of 1:0.1 (VEGF-Trap RS : Fraction) and analyzed by the two charge variant analysis methods. Figure 5 illustrates a panel of electropherograms of VEGF-Trap RS spiked with the VEGF-Trap OFFGEL fractions (5-10) analyzed using iCIEF (Top Panel) and IEF (Bottom panel) assay methods. For each of the OFFGEL fractions spiked into the RS, the corresponding control RS (Unspiked) is overlaid for the IEF and iCIEF assay methods. The overlay of the spiked and unspiked (RS+OFFGEL and RS) samples helps in visualizing and understanding the correlation in pattern of peaks between the IEF and iCIEF assay methods. In each panel, the enhancement of charge species corresponding to the fraction spiked is highlighted using an arrow beginning from the acidic fractions (Figure 5A) to the basic fractions (Figure 5F).

Correlation between the gel based IEF band pattern and the capillary based iCIEF peak pattern was evident from the analysis of OFFGEL fractions. From Figures 4 and 5 (individual fraction analysis together with spiked fractions) it can be inferred that the charge isoform separation achieved by the currently approved IEF gel method is comparable and similar to the pattern of peaks obtained using capillary based iCIEF method for VEGF-Trap.

**Reporting charge variant distribution using the iCIEF method.** The currently approved IEF method for VEGF-Trap charge variant distribution reports the percentage charge variance by grouping bands 3-9 in the IEF gel. The area percentages of bands 3-9 are summed and reported using, e.g., Myoglobin (an independent protein marker) as a guide to identify the band numbers based on the pI of Myoglobin. The current specification acceptance criterion (SPEC) for the IEF method is 82% (Bands 3-9).

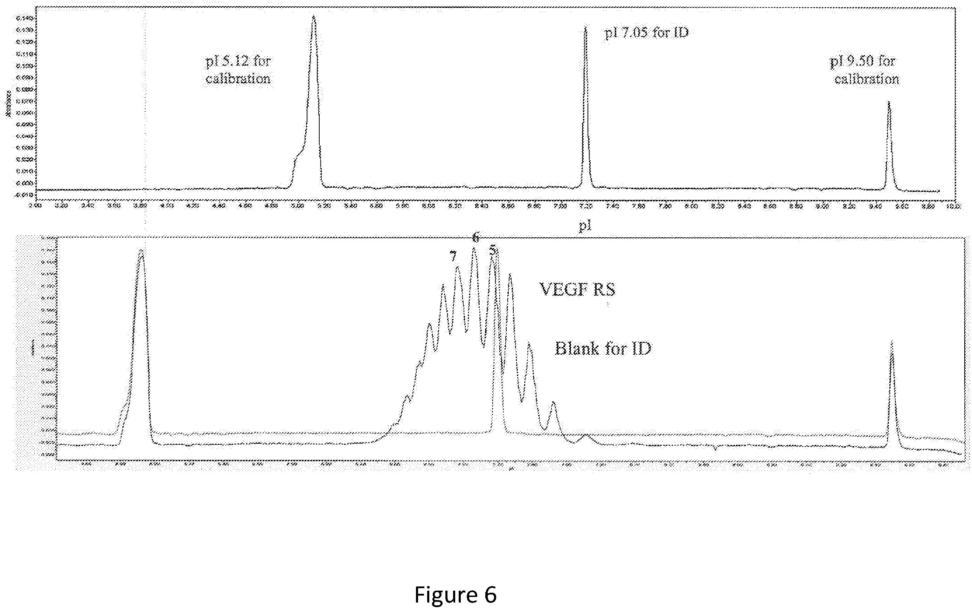

A similar approach was adopted for the new iCIEF assay method where an independent marker from ProteinSimple, pI 7.05 Marker Cat# 102226 is spiked into the iCIEF master mix (2 M urea, 0.35% Methyl Cellulose, 3% 3-10 ampholyte). The iCIEF electropherogram of the marker 7.05 spiked into the master mix is shown in Figure 6 (Top panel), the bracketing pI 5.12 and pI 9.50 markers in the master mix are used for calibration purposes. The bottom panel of Figure 5 shows the iCIEF electropherogram of the VEGF-Trap RS overlaid with the blank containing the spiked 7.05 marker that will be used for identifying Peak 5 in the VEGF-Trap iCIEF sample profile. The marker peak 7.05 migrates at a pI in between peaks 4 and 5 and this will serve to identify the principal peak 6 in the cluster of principal isoforms for VEGF-Trap (Peaks 5, 6 and 7).

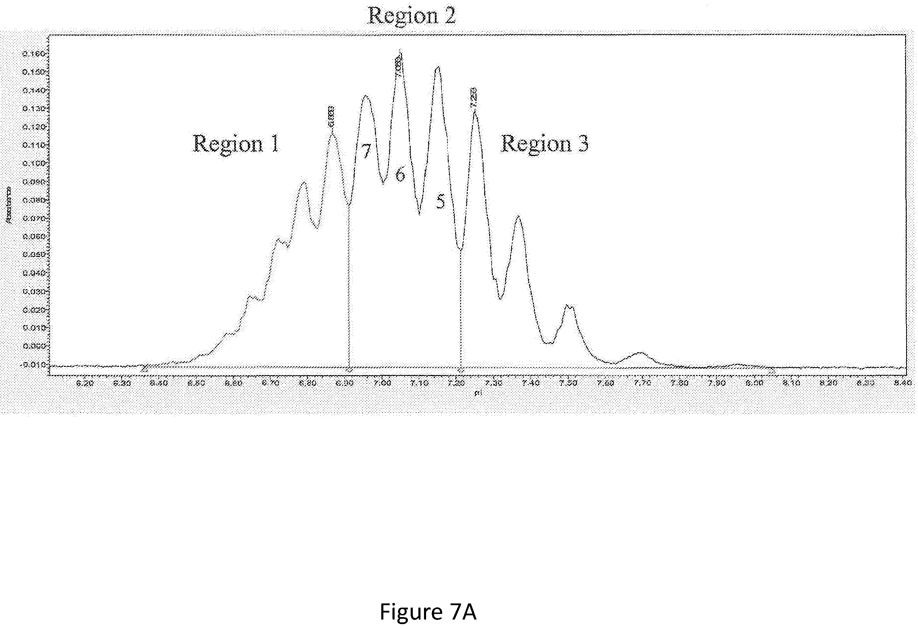

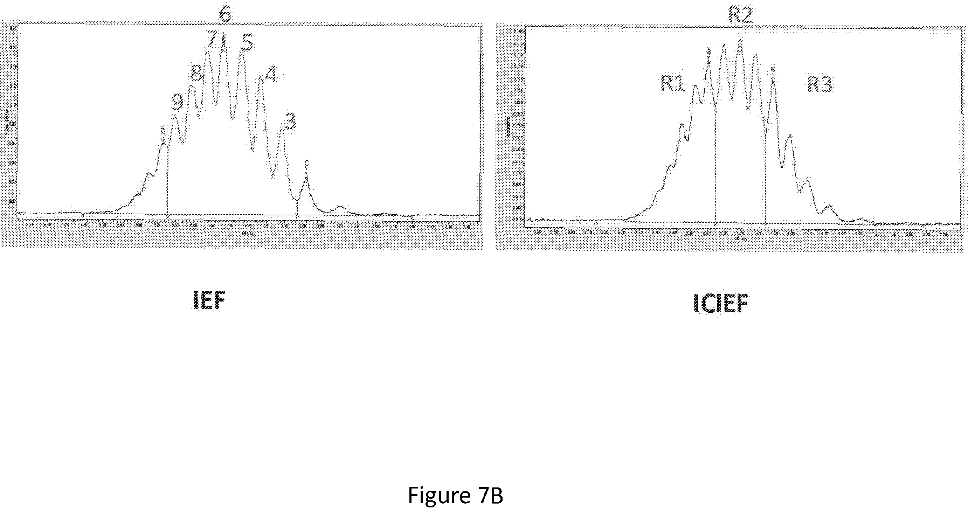

Rather than report peaks 3-9 like the IEF method, the iCIEF method (according to some embodiments) reports the charge heterogeneity of the VEGF-Trap sample in terms of percentages of charge variant isoforms grouped as Region 1 (Acidic), Region 2 (Neutral) and Region 3 (Basic). The cluster of three principal peaks (Peak numbers 5, 6 and 7) in the VEGF-Trap iCIEF electropherogram that migrate around the neutral pI range and which are the most prominent isoforms will be grouped as Region 2 (Neutral). Among the cluster of three peaks, a distinct isoform corresponding to principal peak 5 that migrates to a specific pI is identified using an independent pI 7.05 marker spiked in the blank injection as shown in Figure 7. Region 1 (Acidic) in the VEGF-Trap iCIEF sample is reported as the group of peaks that are relatively acidic compared to the cluster of three principal peaks (Peaks 5, 6 and 7) in the VEGF-Trap electropherogram. Region 3 (Basic) in the VEGF-Trap iCIEF sample is reported as the group of peaks that are relatively Basic compared to the cluster of three principal peaks (Peaks 5, 6 and 7) in the VEGF-Trap electropherogram.

The reporting approach using the Region 1, 2 and 3 offers an advantage of allowing tighter control over the charge variant isoforms by means of monitoring three regions (Regions 1, 2 and 3) as opposed to the traditional IEF gel based method's grouping of bands 3-9. Figure 7B illustrates the differences between IEF reporting and reporting according to embodiments of the iCIEF method of the present disclosure. In addition, the reporting in terms of Regions 1, 2 and 3 offers the iCIEF assay method an unique advantage in its sensitivity to changes in charge variant isoforms much earlier than the traditional approach. This makes the iCIEF assay much more sensitive in its read out and a better stability indicating assay than the previous IEF assay procedure. Table 4 below gives an example of the stability indicating ability of the new grouping approach adopted for the iCIEF assay method as opposed to the traditional 3-9 reporting of the IEF assay. Accelerated VEGF-Trap stability samples were analyzed using the new iCIEF assay by two reporting approaches - Regional grouping and peaks 3-9 similar to the IEF assay method and compared to the historical results from the IEF assay method.

In Table 4 (below), it can be seen that the Region 1, 2 and 3 grouping approach is much more sensitive and indicative of the changes in the charge variant distribution of the VEGF-Trap sample. The IEF method showed a change in overall charge distribution with a decrease of 2 % for the bands 3-9 and this change was comparable to the results from the iCIEF assay method when grouped using the 3-9 peak approach. However, it is evident from Table 4 that for the 25°C accelerated stressed sample of VEGF-Trap, a 5% increase in Region 1 (or acidic variants) and a concomitant decrease of around 5% for Region 3 (Basic variants) was observed using the iCIEF assay. This trend observed in the VEGF-Trap charge distribution in the iCIEF assay is an accurate reflection of the nature of changes to occur in the VEGF-Trap sample based on its structure and complexity of charge pattern attributable to its varying degree of sialylation. The increase in Acidic variants (Region 1 - high degree of sialylated species) of VEGF-Trap sample using the iCIEF assay method under accelerated thermal stress is reflective of possible deamidation coupled with aggregation. On the other hand, grouping using the traditional 3-9 bands by the IEF method masks the subtle changes occurring in the VEGF-Trap charge isoforms and leaves little room to control the different charge species making it not as sensitive a method to detect the subtle changes in the charge heterogeneity of VEGF-Trap sample.

VEGF-Trap has ten glycosylation sites. The glycan chains attached to these sites are branched and each branch may or may not end with the negatively charged sugar monomer, sialic acid. The natural variation in the presence of sialic acid groups at the termini of the glycan chains leads to an ensemble of VEGF-Trap charge variant having a range in net charge. The proportion of these bands varies depending on the abundance of the charged species present. Thus the new reporting approach of grouping the various charge species based on Regions 1 (heavily sialylated), 2 (moderately sialylated) and 3 (least sialylated) makes the iCIEF assay more responsive to the changes that occur in the sialylforms of VEGF-Trap sample.

**Table 4**

| Stress Condition | iCIEF | | | IEF | iCIEF |
|---|---|---|---|---|---|
| | % R1 | % R2 | % R3 | % 3-9 Bands | % 3-9 Peaks |
| VEGF 25°C 1 month | 31.90 | 43.09 | 25.01 | 82.94 | 87.48 |
| VEGF 25°C 3 months | 33.85 | 43.28 | 22.87 | 82.11 | 87.86 |
| VEGF 25°C 6 months | 37.25 | 42.47 | 20.28 | 80.42 | 85.15 |
| Difference (%) | 5.35 | -0.62 | -4.73 | -2.52 | -2.33 |

**Stability Indicating Ability of the iCIEF assay method.** Real time stability samples of VEGF-Trap DP sample (held at 2-8°C) were analyzed using 7 independent time points spanning a time period of 24 months; Table 5 (below) shows the data corresponding to this study. The historical IEF data for these VEGF-Trap samples is provided for reference and compared to VEGF-Trap iCIEF data from regional grouping and 3-9 peak reporting. At the real time storage condition of 2-8°C little to no significant change was observed for the VEGF-Trap sample based on historical IEF data, a similar trend was observed when reported using the iCIEF 3-9 peak approach.

**Table 5 - Real time VEGF-Trap sample analyzed using iCIEF**

| **Condition** | **Time** | **%R1** | **%R2** | **%R3** | **IEF (%3-9)** | **iCIEF (%3-9)** |
|---|---|---|---|---|---|---|
| 5°C | 3 months | 33.67 | 46.49 | 19.84 | 89.21 | 85.45 |
| 5°C | 6 months | 32.90 | 47.14 | 19.96 | 87.09 | 85.26 |
| 5°C | 9 months | 33.20 | 46.99 | 19.81 | 88.78 | 85.23 |
| 5°C | 12 months | 33.71 | 47.05 | 19.24 | 91.37 | 85.12 |
| 5°C | 15 months | 33.71 | 46.80 | 19.49 | 89.92 | 85.05 |
| 5°C | 18 months | 33.89 | 47.00 | 19.11 | 88.27 | 85.25 |
| 5°C | 24 months | 34.14 | 46.87 | 18.99 | 89.79 | 85.03 |

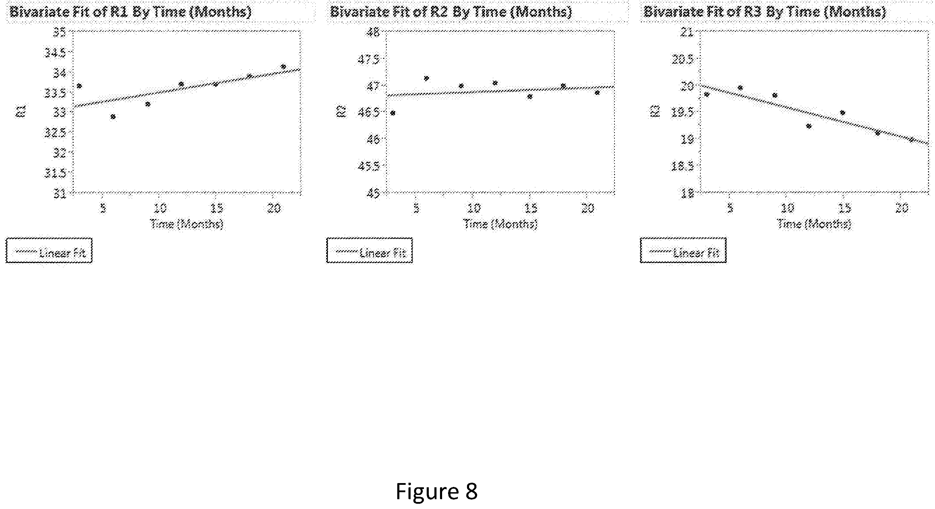

Figure 8 shows the Linear fit of the % Regions 1, 2 and 3 of the VEGF-Trap over the 24 month time period using iCIEF. A small but steady increase in Region 1 with a decrease in Region 3 is evident from the plots.

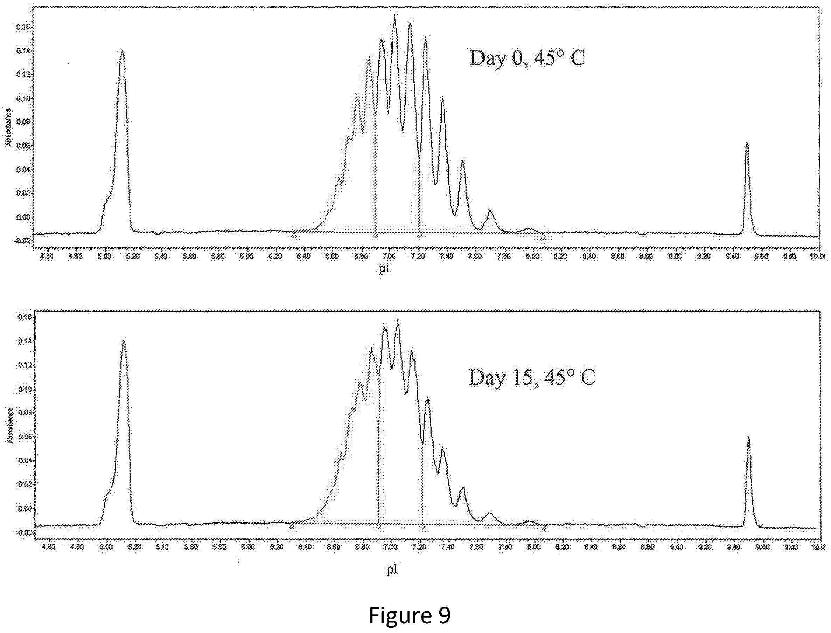

Additional analysis using forcibly degraded VEGF-Trap DS sample was performed using the new iCIEF assay method. For this study, thermally degraded VEGF DS sample diluted and stressed at 45°C for over a period of 15 days was analyzed at 0, 3, 9 and 15 day time points using the iCIEF method (Regional and 3-9). It is evident from Table 6 (below), that a subtle increase in acidic charge variants (Region 1) is observed for the iCIEF assay method when grouped using the Regional approach as compared to the 3-9 peak reporting at a much earlier time point for the forcibly degraded VEGF-Trap sample. While the % 3-9 reporting showed a 2% change in overall charge distribution across the 3-9 peaks, the Region 1 under the same conditions showed a 7% increase while Region 3 showed a concomitant decrease of 8% with time. Figure 9 shows the electropherogram of the forcibly degraded VEGF-Trap sample using the iCIEF assay method; an increase in Acidic species is evident from the profile.

**Table 6 - Percentage distribution of forcibly degraded VEGF-Trap sample analyzed using iCIEF (thermal stress)**

| Stress Condition | iCIEF | | | iCIEF |
|---|---|---|---|---|
| | % R1 | % R2 | % R3 | % 3-9 Peaks |
| VEGF DS 45°C Day 0 | 28.66 | 44.30 | 27.04 | 84.54 |
| VEGF DS 45°C Day 3 | 29.47 | 44.60 | 25.93 | 83.90 |
| VEGF DS 45°C Day 9 | 31.83 | 45.88 | 22.29 | 83.56 |
| VEGF DS 45°C Day 15 | 35.65 | 45.35 | 19.00 | 82.44 |
| Difference (%) | 6.99 | 1.05 | -8.04 | -2.10 |

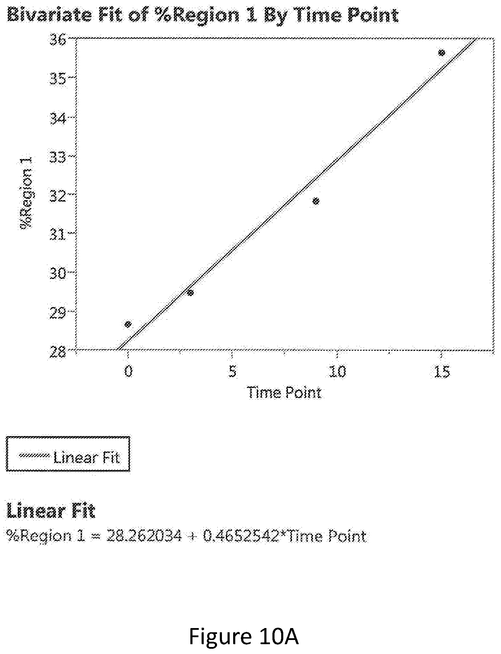

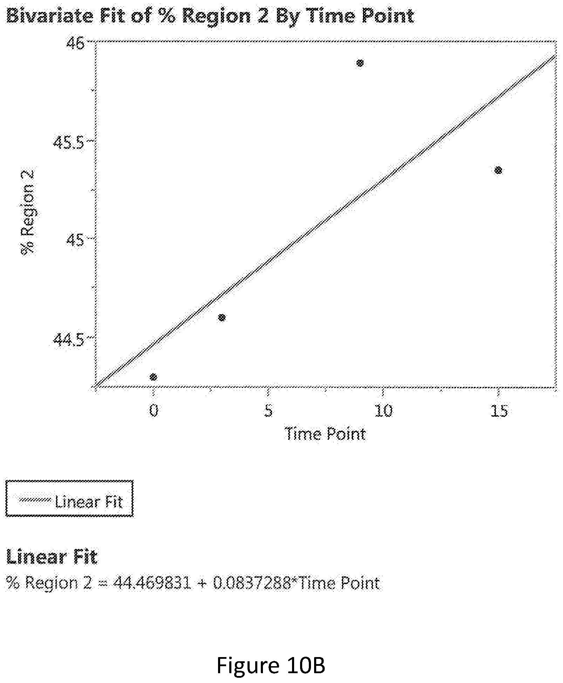

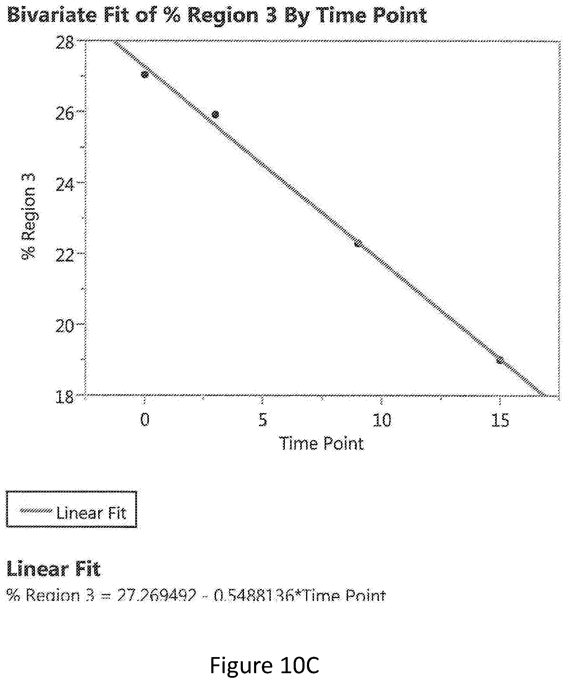

Statistical analysis of the % distribution of the three regions for the VEGF-Trap stress sample was performed by comparing against the respective peak percentage for the VEGF non-stressed sample. Figures 10A-10C show the statistical analysis of the thermally stressed VEGF-Trap sample for the iCIEF data. A statistical significant change was observed for Regions 1 and 3 respectively.

Based on the iCIEF assay method characterization and optimization data, assay parameters were derived and is tabulated in Table 7 (below). These method conditions were assessed for linearity, accuracy, precision and intermediate precision.

**Table 7 - Critical Method Parameters Derived From Development and Optimization Experiments**

| | |
|---|---|
| Urea | 2M |
| Methylcellulose | 0.35% |
| ampholyte 3-10 | 3% |
| VEGF-Trap | 1.0 mg/mL |
| Pre-focusing | 1 min @ 1500 V |
| Focusing | 7 min @ 3000 V |

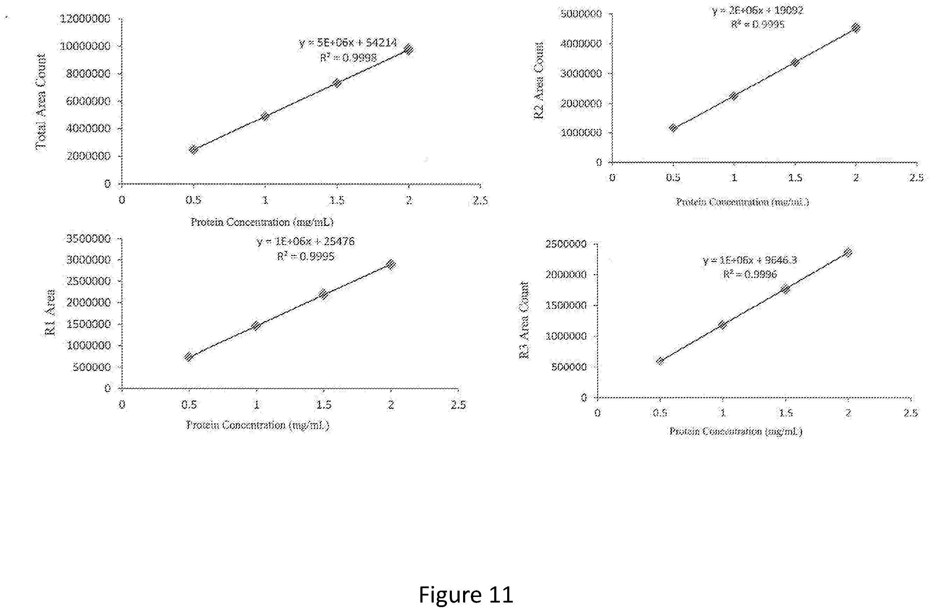

***iCIEF Assay Method Qualification.*** Linearity of iCIEF assay method. Method linearity was evaluated by a single analyst. Sample solutions were prepared using VEGF-Trap reference standard at varying protein concentrations of 0.5 mg/mL, 1.0 mg/mL, 1.5 mg/mL and 2.0 mg/mL. In this experiment, the protein concentration in the sample matrix was varied from 0.5 to 2 mg/mL while keeping other matrix components constant at 3% ampholyte 3-10 and 0.35% methylcellulose. The focusing time was also kept constant at 1+7 minutes. Table 8 summarizes the percentage distribution of Regions 1, 2 and 3 for the VEGF-Trap sample across the linear range of 0.5 to 2.0 mg/mL. The Linearity plot of concentration as a function of Area counts for the VEGF-Trap sample is provided in Figure 11.

**Table 8 - Linearity of the iCIEF assay method**

| **VEGF-Trap RS (mg/mL)** | **% R1** | **% R2** | **% R3** |
|---|---|---|---|
| 0.5 | 29.51 | 46.44 | 24.05 |
| 0.5 | 29.79 | 46.38 | 23.84 |
| 1 | 29.66 | 45.88 | 24.46 |
| 1 | 30.03 | 45.94 | 24.02 |
| 1.5 | 30.27 | 45.83 | 23.91 |
| 1.5 | 29.68 | 45.95 | 24.37 |
| 2 | 29.54 | 46.31 | 24.15 |
| 2 | 29.57 | 46.26 | 24.17 |

The assay demonstrated acceptable linearity over a protein concentration range of 0.5 mg/mL to 2.0 mg/mL with R2 > 0.99 based on the regression analysis. In addition, the isoform distribution remained consistent over this same concentration range. This indicates that the assay is capable of providing consistent results in both peak area and isoform distribution over the protein concentration range of 0.5 to 2.0 mg/mL.

Accuracy of iCIEF assay method. Method accuracy was evaluated based on dilutional proportionality using the linearity data by comparing to Nominal concentration of 1.0 mg/mL. The dilutional recovery based on Linearity data is shown in Table 9 below. Percent recovery was calculated using = (Measured area percentage / Nominal area percentage) x 100%.

**Table 9 - Accuracy of the VEGF-Trap iCIEF assay based on dilutional proportionality**

| | % Average | | | % Recovery | | |
|---|---|---|---|---|---|---|
| VEGF-Trap RS (mg/mL) | % R1 | % R2 | % R3 | % R1 | % R2 | % R3 |
| 0.5 | 29.65 | 46.41 | 23.95 | 99.35 | 101.09 | 98.78 |
| 1 | 29.85 | 45.91 | 24.24 | Nominal | | |
| 1.5 | 29.98 | 45.89 | 24.14 | 100.44 | 99.96 | 99.59 |
| 2 | 29.56 | 46.29 | 24.16 | 99.03 | 100.82 | 99.67 |

The recovery based on dilutional proportionality in the range of 0.5 to 2.0 mg/mL protein concentration for the VEGF-Trap sample was within 98%-101% for the three regions.

Intermediate Precision Analysis of iCIEF method. Intermediate precision was evaluated by two separate analysts (A, B) using their respective reagent preparations and using two iCE3 charge variant analyzer instruments across four days for the VEGF-Trap RS sample. The results of the analysis are listed in Table 10 below.

**Table 10 - Results of Intermediate Precision analysis**

| Analyst | %Region 1 | %Region 2 | %Region 3 |
|---|---|---|---|
| A | 29.44 | 45.87 | 24.69 |
| | 30.53 | 45.08 | 24.39 |
| | 29.71 | 45.83 | 24.46 |
| A | 29.91 | 45.98 | 24.11 |
| | 29.50 | 46.18 | 24.31 |
| | 29.76 | 46.14 | 24.10 |
| B | 28.78 | 46.65 | 24.57 |
| | 29.40 | 46.22 | 24.38 |
| | 29.61 | 45.85 | 24.54 |
| B | 29.56 | 46.10 | 24.34 |
| | 30.01 | 45.85 | 24.15 |
| | 29.50 | 45.89 | 24.61 |
| Overall Average | 29.64 | 45.97 | 24.39 |
| Std Dev | 0.42 | 0.37 | 0.20 |
| % RSD | 1.40 | 0.80 | 0.81 |

The proposed VEGF-Trap iCIEF test method demonstrated acceptable precision when executed by different analysts using different reagent preparations. The overall % RSD was calculated and was within an RSD of 2% for all three Regions.

Robustness of iCIEF assay method. Several elements of assay method robustness were evaluated by various experiments. These experiments are listed in Table 11.

**Table 11 - Summary of Method Robustness Experiments**

| **Robustness Experiment** | **Performance Evaluated** |
|---|---|
| Prepared solution stability | Consistency of isoform distribution over 24 hours compared to time T=O |
| Evaluation other samples of ampholyte 3-10 | Consistency in overall profile and % distribution across the three Regions |
| Evidence of stability indication | Effect of stress exposure on isoform distribution |

Prepared Solution Stability in machine. Solution stability was evaluated by preparing a sample of reference standard in the sample matrix and analyzing the sample using iCIEF. The sample was stored in the iCE3 instrument after analysis at 10°C in the matrix consisting of 3% ampholyte 3-10, 0.35% methylcellulose and 2 M urea. The sample was analyzed again approximately 24 hours later. The isoform distribution for each analysis is presented below in Table 12 below.

**Table 12 - Results of prepared iCIEF sample Stability in machine**

| Sample Name | %Region 1 | %Region 2 | %Region 3 |
|---|---|---|---|
| VEGF RS at time 0 injection 1 | 29.49 | 45.72 | 24.80 |
| VEGF RS at time 0 injection 2 | 29.88 | 45.62 | 24.50 |
| VEGF RS at time 24 hours injection 1 | 29.52 | 45.92 | 24.56 |
| VEGF RS at time 24 hours injection 2 | 29.95 | 45.74 | 24.31 |
| % Difference | 0.46 | 0.30 | 0.49 |

The absolute difference between the sample analyzed at T=O and again at T=24 hours was calculated based on the range (Maximum and Minimum) observed at each time point. The absolute difference was equal to or less than 0.5% for the three Regions. This indicates the sample is stable in the matrix for up to 24 hours when stored at 10°C in the iCE3 Charge Variant analyzer.

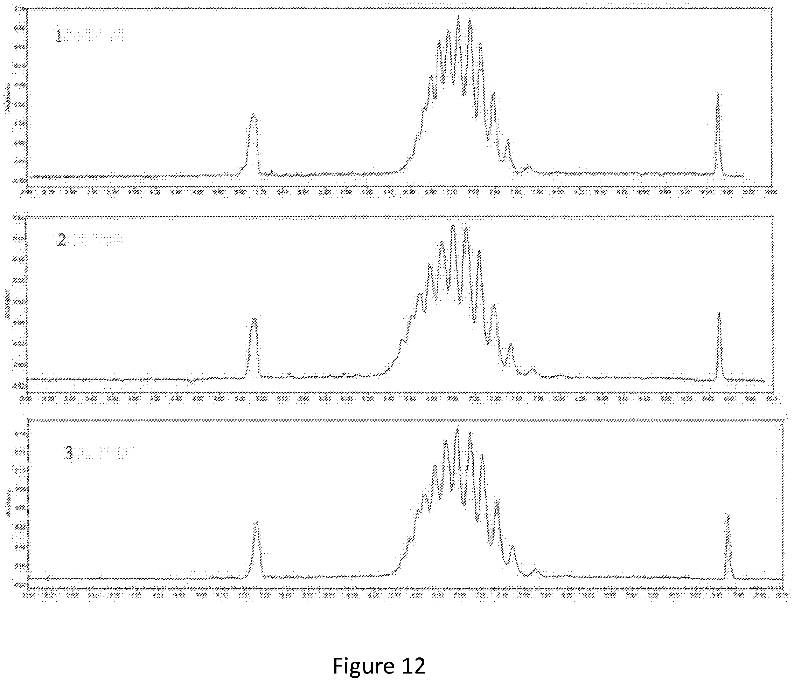

Evaluation of Samples of ampholyte (3-10). Several samples of the 3-10 ampholyte from one source were analyzed. The overall charge variant profile of the VEGF-Trap sample analyzed using different samples were comparable. Minor differences in electropherogram profile in terms of peak pattern were observed in Region 1 for some ampholyte samples however, the percentage distribution were similar and within assay variability. Representative electropherograms from three samples of ampholyte are shown in Figure 12.

### Example - Analysis of historical VEGF-Trap release samples (DS, FDS and DSI) using the new iCIEF assay method

In order to further establish the robustness of the iCIEF assay method, a total of 37 unique historical VEGF-Trap samples that are not related in their genealogy were analyzed using iCIEF using two samples of ampholytes. The analysis included, 15 VEGF-Trap DSI (Drug Substance Intermediate, Aqueous buffered solution, pH 6.2, comprising 5 mM sodium phosphate, 5 mM sodium citrate and 100 mM sodium chloride), 10 VEGF-Trap DS samples (Drug substance SPEC C701, Aqueous buffered solution, pH 6.2, containing 10 mM sodium phosphate) and 12 VEGF-Trap FDS samples (Formulated Drug Substance, SPEC C713 Aqueous buffered solution, pH 6.2, comprising 10 mM, Sodium phosphate, 40 mM sodium chloride, 0.03% (w/v) polysorbate 20 and 5% (w/v) surcrose). These VEGF-Trap samples were analyzed using the iCIEF assay method.

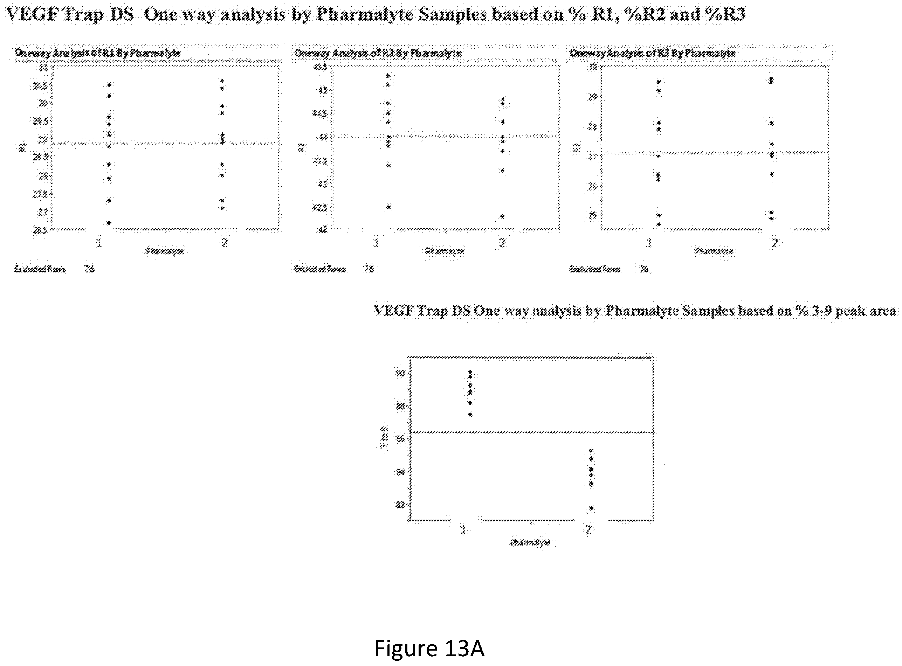

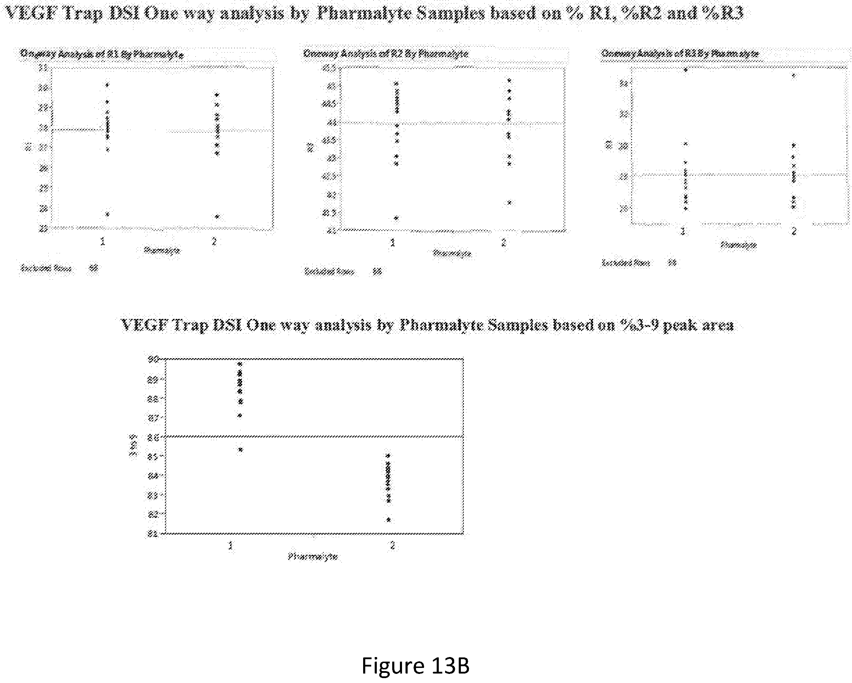

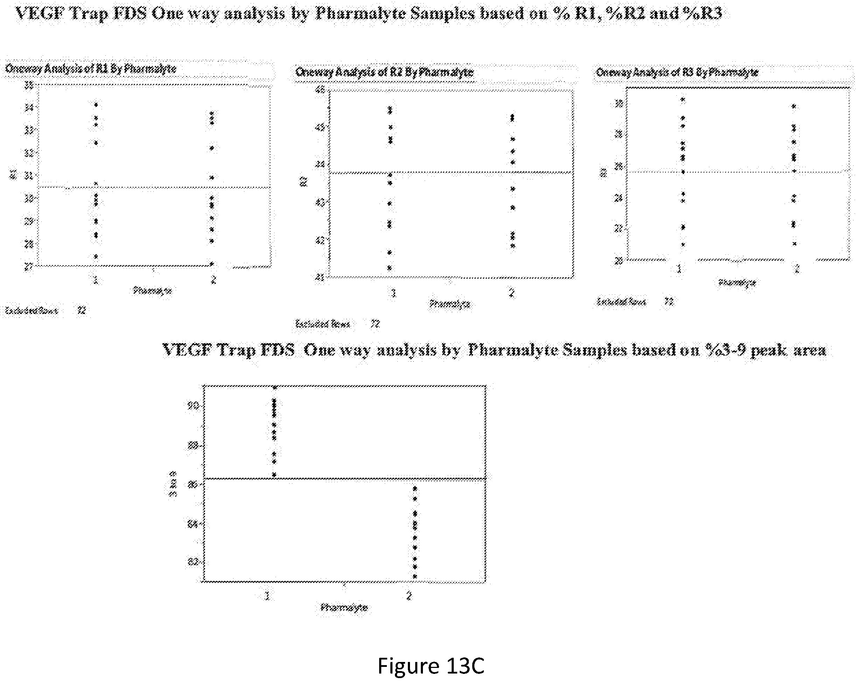

Ampholytes are a mixture of different homologues of amphoteric compounds with a spectrum of isoelectric points between 3 and 10 that help establish the pH gradient under the influence of the electric field. The ampholyte 3-10 used in the iCIEF assay method was purchased from one source which are typically produced in batches. Based on the recommendation from the vendor together with our working knowledge on the iCIEF assay for other proteins, slight variations between the different samples has been observed and is inevitable. Hence, in order to establish the robustness of the new iCIEF assay across the different ampholyte sample, two samples of ampholytes were analyzed. The VEGF samples from DS, DSI and FDS products stage were analyzed using the proposed Regional grouping approach (R1, R2 and R3) and also based on 3-9 peak grouping similar to the IEF assay method. Figures 13A-13C show the statistical analysis of the ampholyte samples as a function of % Region 1, 2 and 3 and peaks 3-9 for VEGF-Trap DS, DSI and FDS samples. The data corresponding to the 37 VEGF-Trap samples is provided in Table 13 for one of the ampholyte samples. Tables 14-16 provide the complete data set for 37 samples.

**Table 13 - Historical VEGF-Trap DSI, DS and FDS Samples analyzed using iCIEF assay procedure**

| **VEGF-Trap DSI Sample** | **%R1** | **%R2** | **%R3** | **% 3 to 9 peaks** |
|---|---|---|---|---|
| 1 | 29.7 | 43.6 | 26.7 | 82.7 |
| 2 | 28.5 | 43.6 | 28.0 | 83.5 |
| 3 | 28.0 | 44.3 | 27.7 | 84.2 |
| 4 | 28.1 | 44.2 | 27.8 | 83.9 |
| 5 | 28.7 | 44.9 | 26.4 | 84.6 |
| 6 | 27.6 | 44.2 | 28.2 | 84.0 |
| 7 | 23.7 | 41.8 | 34.6 | 81.7 |
| 8 | 26.8 | 43.1 | 30.1 | 82.9 |
| 9 | 28.5 | 45.2 | 26.4 | 85.0 |
| 10 | 27.2 | 44.1 | 28.7 | 84.0 |
| 11 | 28.5 | 45.2 | 26.4 | 85.0 |
| 12 | 27.9 | 44.3 | 27.8 | 84.4 |
| 13 | 28.0 | 43.7 | 28.3 | 83.7 |
| 14 | 27.8 | 42.9 | 29.3 | 83.3 |
| 15 | 29.2 | 44.7 | 26.1 | 84.3 |
| 16 | 29.0 | 44.0 | 27.0 | 84.2 |
| 17 | 28.9 | 43.7 | 27.4 | 83.8 |
| 18 | 30.6 | 44.3 | 25.1 | 84.1 |
| 19 | 29.7 | 43.9 | 26.4 | 83.8 |
| 20 | 29.9 | 43.7 | 26.4 | 83.3 |
| 21 | 29.1 | 43.9 | 27.1 | 84.1 |
| 22 | 27.1 | 44.8 | 28.1 | 85.3 |
| 23 | 30.4 | 44.7 | 24.9 | 84.8 |
| 24 | 28.3 | 43.7 | 28.1 | 83.2 |
| 25 | 27.3 | 43.3 | 29.5 | 83.2 |
| 26 | 28.0 | 42.3 | 29.6 | 81.8 |
| 27 | 27.2 | 45.2 | 27.6 | 85.8 |
| 28 | 30.1 | 43.4 | 26.5 | 84.1 |
| 29 | 29.2 | 44.1 | 26.7 | 84.0 |
| 30 | 29.8 | 43.4 | 26.8 | 83.3 |
| 31 | 29.7 | 44.7 | 25.7 | 84.6 |
| 32 | 32.3 | 45.3 | 22.4 | 84.5 |
| 33 | 33.4 | 44.4 | 22.2 | 83.8 |
| 34 | 33.6 | 45.2 | 21.1 | 84.5 |
| 35 | 31.0 | 45.2 | 23.8 | 85.3 |
| 36 | 33.8 | 42.1 | 24.1 | 81.3 |
| 37 | 29.2 | 42.2 | 28.6 | 82.2 |
| 38 | 28.2 | 41.9 | 29.9 | 81.8 |
| 39 | 28.7 | 42.9 | 28.4 | 82.8 |

**Table 14 - DSI lots analyzed by Pharmalyte Samples**

| **VEGF Trap DSI Samples** | **%R1** | **%R2** | **%R3** | **% 3 to 9 peaks** |
|---|---|---|---|---|
| 1 | 30.2 | 43.1 | 26.7 | 87.9 |
| 2 | 27.8 | 44.4 | 27.8 | 88.8 |
| 3 | 28.0 | 44.5 | 27.6 | 88.9 |
| 4 | 27.9 | 44.4 | 27.8 | 88.7 |
| 5 | 28.8 | 44.8 | 26.4 | 85.3 |
| 6 | 27.5 | 44.3 | 28.2 | 87.1 |
| 7 | 23.7 | 41.4 | 34.9 | 89.7 |
| 8 | 26.9 | 42.9 | 30.2 | 88.4 |
| 9 | 28.5 | 45.1 | 26.4 | 89.3 |
| 10 | 27.6 | 43.9 | 28.4 | 88.7 |
| 11 | 28.3 | 44.9 | 26.8 | 88.3 |
| 12 | 28.1 | 44.6 | 27.3 | 87.8 |
| 13 | 28.2 | 43.7 | 28.1 | 87.8 |
| 14 | 27.6 | 43.5 | 28.9 | 87.9 |
| 15 | 29.3 | 44.7 | 26.0 | 89.2 |

**Table 15 - DS lots analyzed by Pharmalyte Samples**

| **VEGF Trap DS Samples** | **%R1** | **%R2** | **%R3** | **% 3 to 9 peaks** |
|---|---|---|---|---|
| 1 | 28.8 | 44.7 | 26.4 | 89.3 |
| 2 | 29.4 | 44.3 | 26.3 | 89.2 |
| 3 | 29.2 | 44.5 | 26.2 | 88.9 |
| 4 | 27.3 | 43.4 | 29.2 | 88.2 |
| 5 | 29.6 | 44.0 | 26.4 | 88.9 |
| 6 | 28.3 | 43.8 | 27.9 | 88.8 |
| 7 | 26.7 | 45.3 | 28.1 | 89.8 |
| 8 | 30.2 | 45.1 | 24.7 | 90.1 |
| 9 | 30.5 | 44.5 | 25.0 | 89.3 |
| 10 | 29.1 | 43.9 | 27.0 | 89.3 |
| 11 | 27.9 | 42.5 | 29.5 | 87.5 |

**Table 16 - FDS lots analyzed by Pharmalyte Samples**

| **VEGF Trap FDS Samples** | **%R1** | **%R2** | **%R3** | **% 3 to 9 peaks** |
|---|---|---|---|---|
| 1 | 27.5 | 45.0 | 27.5 | 90.0 |
| 2 | 30.0 | 43.5 | 26.5 | 88.7 |
| 3 | 29.1 | 43.7 | 27.2 | 89.1 |
| 4 | 30.2 | 43.0 | 26.7 | 88.4 |
| 5 | 29.8 | 44.6 | 25.6 | 89.8 |
| 6 | 32.5 | 45.4 | 22.1 | 90.3 |
| 7 | 33.3 | 44.7 | 22.0 | 89.6 |
| 8 | 33.6 | 45.4 | 21.0 | 90.9 |
| 9 | 30.7 | 45.5 | 23.8 | 90.1 |
| 10 | 34.2 | 41.7 | 24.2 | 87.2 |
| 11 | 29.0 | 42.4 | 28.6 | 87.2 |
| 12 | 28.4 | 41.3 | 30.3 | 86.5 |
| 13 | 28.5 | 42.5 | 29.1 | 87.6 |

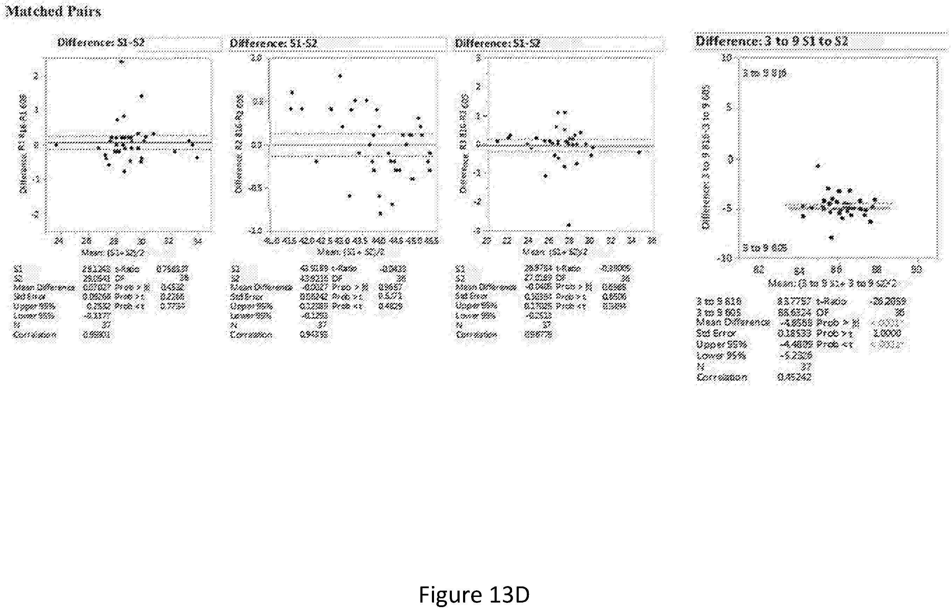

A Matched Pair analysis (Figure 13D) was performed based on the data collected for samples of ampholyte for the Region 1, 2 and 3 and 3-9 grouping approach. The data from the matched pairs analysis was used to compare the means between the two ampholytes and to assess any difference in reporting of the assay that may be observed due to inherent differences in ampholyte samples. Based on the data, it can be inferred that the maximum observed Mean difference between samples for the three regions R1, R2 and R3 is less than 0.1% when reported in terms of Regions. In addition, based on the p- value it can be concluded that the % distribution across Region 1, 2 and 3 are not statistically significant between the two ampholyte samples using the Regional approach.

However, when the VEGF-Trap data set for DS, DSI and FDS samples was analyzed using the 3-9 peaks grouping approach similar to the IEF assay method, a statistical significant difference is noticed between the some ampholyte samples. For example, between some samples, a mean difference as high as 4.8% when reported in terms of peaks 3-9 for the iCIEF assay method. Figures 13A-13D show the iCIEF profiles obtained using the different ampholyte samples and it is evident from the images that the variability observed between ampholyte samples is restricted to the acidic region and by grouping Regions 1, 2 and 3 that variability is masked. This makes the regional approach of reporting for the iCIEF assay a robust and reproducible approach.

The data from the DSI, DS and FDS sample analysis using the ampholyte samples based on %Regions 1, 2 and 3 grouping makes the iCIEF assay a more robust assay method.

Accordingly, the iCIEF system, methods and devices presented in this disclosure quantify the charge variant profile of VEGF-Trap drug substance, drug substance intermediate, formulated drug substance, and drug product. Such embodiments may serve to replace the currently approved gel based IEF method for charge heterogeneity analysis of VEGF-Trap.

The VEGF-Trap charge variants fractionated using OFFGEL 3100 fractionator enabled demonstration of a correlation between the peaks obtained in the capillary based iCIEF assay method to the bands resolved in the gel based IEF assay procedure. By analyzing the OFFEGEL electrophoresed VEGF-Trap fractions individually and through spike in studies a direct comparison of individual iCIEF peaks to IEF bands of the VEGF-Trap charge variants was achieved. The studies confirmed that the new iCIEF assay procedure is capable of resolving all the charge variant isoforms previously resolved using the gel based IEF method with equal and a more precise manner.

Accordingly, some embodiments of this reporting approach based on percentage distribution of Regions 1, 2 and 3 disclosed herein allow for control over all VEGF-Trap isoforms and makes the assay more sensitive, enabling it to be a robust stability indicating assay.

Capillary tubes for use with the iCEF methods are also described herein. In general, the iCIEF capillary tube is configured for use in a charge variant analysis of VEGF-Trap and includes a capillary tube configured to receive a protein, and configured with a mixture of carrier ampholyte, methylcellulose, and a stabilizing additive. The capillary tube may also include a fluorocarbon coating.

In some embodiments, an iCIEF kit configured for use in a charge variant analysis of VEGF-Trap is provided and includes one or more capillary tubes configured to receive a protein, and configured with a mixture of carrier ampholyte, methylcellulose, and a stabilizing additive, wherein the capillary tube includes a fluorocarbon coating.

While various inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, amounts, percentages, concentrations, dimensions, materials, and configurations described herein are meant to be an example and that the actual parameters, amounts, percentages, concentrations, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only. Inventive embodiments disclosed herein may be practiced otherwise than as specifically described and claimed. Inventive embodiments of the present disclosure also include individual features, system, article, material, kit, and methods described herein. In addition, any combination of two or more such features, systems, articles, materials, kits, and methods are also inventive (if such are not mutually inconsistent). Some embodiments may be distinguishable from the prior art for specifically lacking one or more features/elements/functionality (i.e., claims directed to such embodiments may include negative limitations).

In addition, as noted, various inventive concepts may be embodied as one or more methods. The acts performed as part of the method may be ordered in any suitable way. Accordingly, embodiments may be constructed in which acts are performed in an order different than illustrated, which may include performing some acts simultaneously, even though shown as sequential acts in illustrative embodiments.

Any and all references to publications or other documents presented anywhere in the present application, are herein incorporated by reference in their entirety. Moreover, all definitions, as defined and used herein, should be understood to control over dictionary definitions, definitions in documents incorporated by reference, and/or ordinary meanings of the defined terms.

The invention further provides the following numbered embodiments.
1. A method for analyzing charge variants of vascular endothelial growth factor VEGF-Trap comprising:
   loading a protein sample onto a separation capillary comprising a mixture of at least a carrier ampholyte, methylcellulose, and a stabilizing additive;
   applying a first voltage for a first predetermined period of time such that the carrier ampholyte forms a pH gradient within the capillary;
   applying a second voltage for a second predetermined period of time to focus the migration of charge variants of the protein within the capillary such that the overall charge of the variants is neutral; and
   detecting and quantifying charge variants of the protein.
2. The method of embodiment 1, wherein:
   detecting and quantifying charge variants comprises:
   measuring the absorbance for a plurality of charge variant isoforms;
   segregating the plurality of charge variant isoforms into isolated regions comprising at least a first acid/acidic region (R1), a second neutral region (R2), and a third base/basic region (R3); and
   determining a percentage of charge variant isoforms falling within in each of regions R1, R2 and R3.
3. The method of embodiment 1, wherein both detecting and quantifying of charge variants are performed.
4. The method of embodiment 1, wherein detection of charge variants occurs at a wavelength of approximately 280 nm.
5. The method of embodiment 1, wherein the concentration of protein loaded onto the capillary tube is approximately 2.0 mg/ml.
6. The method of embodiment 1, wherein stabilizing additive comprises urea.
7. The method of embodiment 5, wherein the amount of urea in the mixture comprises approximately 2 M.
8. The method of embodiment 1, wherein the mixture includes approximately 0.35% methylcellulose.
9. The method of embodiment 1, wherein the first voltage comprises 1500 V.
10. The method of embodiment 1, wherein the first predetermined time is approximately 1 minute.
11. The method of embodiment 1, wherein the second voltage comprises 3000 V.
12. The method of embodiment 1, wherein the second predetermined time is approximately 7 minute.
13. The method of embodiment 1, wherein the concentration of protein loaded onto the capillary tube is between approximately 1.0 and 8.0 mg/ml, or any intervening range.
14. The method of embodiment 6, wherein the amount of urea in the mixture is greater than 0M and less than approximately 8M, or any intervening range.
15. The method of embodiment 1, wherein the mixture includes between approximately 0.01% and approximately 0.35% methylcellulose, or any intervening range.
16. The method of embodiment 1, wherein the first voltage is between approximately 1 V and about 3000 V, or any intervening range.
17. The method of embodiment 1, wherein the first predetermined time is between approximately 1 second and approximately 5 minutes, or any intervening range.
18. The method of embodiment 1, wherein the second voltage is between about 1 V and approximately 6000 V, or any intervening range.
19. The method of embodiment 1, wherein the second predetermined time is between approximately 1 minute and approximately 14 minute, or any intervening range.
20. An iCIEF capillary tube configured for use in a charge variant analysis of VEGF-Trap comprising:
   a capillary tube configured to receive a protein, and configured with a mixture of carrier ampholyte, methylcellulose, and a stabilizing additive,
   wherein the capillary tube includes a fluorocarbon coating.
21. The capillary tube of embodiment 20, wherein the components thereof conform to any one and/or another of embodiments 2-19.
22. An iCIEF kit configured for use in a charge variant analysis of VEGF-Trap comprising one or more capillary tubes according to any of embodiments 19-20.
23. A system for performing the methods according to any one or another of embodiments 1-19.

## Claims

1. An image capillary isoelectric focusing (iCIEF) method for analyzing charge variants of a vascular endothelial growth factor trap (VEGF-Trap) protein comprising:
loading a protein sample comprising the VEGF-Trap protein onto a separation capillary tube comprising a mixture of at least a carrier ampholyte, methylcellulose, and a stabilizing additive;
applying a first voltage for a first predetermined period of time such that the carrier ampholyte forms a pH gradient within the separation capillary tube;
applying a second voltage for a second predetermined period of time to focus the migration of charge variants of the VEGF-Trap protein within the separation capillary tube such that the overall charge of the variants is neutral; and
detecting and quantifying charge variants of the VEGF-Trap protein comprising:
(i) measuring an absorbance for a plurality of charge variant isoforms;
(ii) segregating the plurality of charge variant isoforms into isolated regions comprising at least an acidic region (R1), a neutral region (R2), and a basic region (R3); and
(iii) determining a percentage of charge variant isoforms falling within each of regions R1, R2 and R3.

2. The method of claim 1, wherein detection of charge variants occurs at a wavelength of 280 nm.

3. The method of claim 1, wherein the concentration of VEGF-Trap protein loaded onto the separation capillary tube is 2.0 mg/ml.

4. The method of claim 1, wherein stabilizing additive comprises urea.

5. The method of claim 4, wherein the amount of urea in the mixture comprises 2 M or wherein the amount of urea in the mixture is greater than 0M and less than 8M, or any intervening range.

6. The method of claim 1, wherein the mixture includes 0.35% methylcellulose.

7. The method of claim 1, wherein:
a) the first voltage comprises 1500 V;
b) the first predetermined period of time is 1 minute;
c) the second voltage comprises 3000 V; or
d) the second predetermined period of time is 7 minutes.

8. The method of claim 1, wherein:
a) the concentration of VEGF-Trap protein loaded onto the separation capillary tube is between 1.0 and 8.0 mg/ml, or any intervening range;
b) the mixture includes between 0.01% and 0.35% methylcellulose, or any intervening range;
c) the first voltage is between 1 V and 3000 V, or any intervening range;
d) the first predetermined period of time is between 1 second and 5 minutes, or any intervening range;
e) the second voltage is between 1 V and 6000 V, or any intervening range; or
f) the second predetermined period of time is between 1 minute and 14 minutes, or any intervening range.

9. The method of claim 1, wherein the VEGF-Trap protein comprises the sequence of SEQ ID NO: 1.

10. The method of claim 1, wherein the neutral region (R2) comprises three principal peaks identified relative to an independent marker peak.

11. The method of claim 1, wherein:
a) the mixture comprises 2M urea, 0.35% methylcellulose, and 3% ampholyte having a pI of 3-10;
b) the first voltage comprises 1500V and the first predetermined period of time is 1 minute;
c) the second voltage comprises 3000 V and the second predetermined period of time is 7 minutes; and
d) the VEGF-Trap protein comprises the sequence of SEQ ID NO: 1.

12. The method of claim 11, wherein the concentration of VEGF-Trap protein loaded onto the separation capillary tube is 1.0 mg/mL.

13. The method of any one of claims 1-12, wherein the capillary tube includes a fluorocarbon coating.

14. The method of any one of claims 1-13, wherein the charge variants of the VEGF-Trap protein comprise sialylated VEGF-Trap proteins.

15. The method of any one of claims 1-14, wherein sensitivity of the method to changes in charge variant isoforms is increased compared to a method of grouping charge variant isoforms within bands 3 to 9 of an isoelectric focusing (IEF) assay.
